# EUROPEAN PATENT SPECIFICATION

(11) **EP 1 401 439 B1**
(45) Date of publication and mention of the grant of the patent: **19.04.2006**
(21) Application number: 02744604.6
(22) Date of filing: 25.06.2002
(51) Int. Cl.: A61K 31/439, A61P 25/28

(54) **USE OF BICYCLO COMPOUNDS FOR TREATING ALZHEIMER'S DISEASE**
VERWENDUNG VON BICYKLISCHEN VERBINDUNGEN FÜR DIE BEHANDLUNG VON ALZHEIMER
UTILISATION DE COMPOSES BICYCLIQUES DANS LE TRAITEMENT DE LA MALADIE D'ALZHEIMER

(30) Priority: 25.06.2001 US 300671 P
(43) Date of publication of application: 31.03.2004
(73) Proprietor: ELAN PHARMACEUTICALS, INC., South San Francisco, CA 94080 (US); Pharmacia & Upjohn Company LLC, Kalamazoo, MI 49001 (US)
(72) Inventor: BECK, James P., Kalamazoo, MI 49009 (US)
(74) Representative: Adam, Holger
(86) International application number: PCT/US2002/020054
(87) International publication number: WO 2003/000261

(56) References cited:
- WO-A-96/30372
- US-A- 5 846 978
- SKOVRONSKY D M ET AL: "beta-Secretase revealed: starting gate for race to novel therapies for Alzheimer's disease" TRENDS IN PHARMACOLOGICAL SCIENCES, ELSEVIER TRENDS JOURNAL, CAMBRIDGE, GB, vol. 21, no. 5, May 2000 (2000-05), pages 161-163, XP004198178 ISSN: 0165-6147

## Description

### Field of the Invention

The present invention relates to the treatment of Alzheimer's disease and other similar diseases, and more specifically to the use of compounds that inhibit beta-secretase, an enzyme that cleaves amyloid precursor protein to produce A beta peptide, a major component of the amyloid plaques found in the brains of Alzheimer's sufferers, in such methods.

### Background of the Invention

Alzheimer's disease (AD) is a progressive degenerative disease of the brain primarily associated with aging. Clinical presentation of AD is characterized by loss of memory, cognition, reasoning, judgment, and orientation. As the disease progresses, motor, sensory, and linguistic abilities are also affected until there is global impairment of multiple cognitive functions. These cognitive losses occur gradually, but typically lead to severe impairment and eventual death in the range of four to twelve years.

Alzheimer's disease is characterized by two major pathologic observations in the brain: neurofibrillary tangles and beta amyloid (or neuritic) plaques, comprised predominantly of an aggregate of a peptide fragment know as A beta. Individuals with AD exhibit characteristic beta-amyloid deposits in the brain (beta amyloid plaques) and in cerebral blood vessels (beta amyloid angiopathy) as well as neurofibrillary tangles. Neurofibrillary tangles occur not only in Alzheimer's disease but also in other dementia-inducing disorders. On autopsy, large numbers of these lesions are generally found in areas of the human brain important for memory and cognition.

Smaller numbers of these lesions in a more restricted anatomical distribution are found in the brains of most aged humans who do not have clinical AD. Amyloidogenic plaques and vascular amyloid angiopathy also characterize the brains of individuals with Trisomy 21 (Down's Syndrome), Hereditary Cerebral Hemorrhage with Amyloidosis of the Dutch-Type (HCHWA-D), and other neurodegenerative disorders. Beta-amyloid is a defining feature of AD, now believed to be a causative precursor or factor in the development of disease. Deposition of A beta in areas of the brain responsible for cognitive activities is a major factor in the development of AD. Beta-amyloid plaques are predominantly composed of amyloid beta peptide (A beta, also sometimes designated betaA4). A beta peptide is derived by proteolysis of the amyloid precursor protein (APP) and is comprised of 39-42 amino acids. Several proteases called secretases are involved in the processing of APP.

Cleavage of APP at the N-terminus of the A beta peptide by beta-secretase and at the C-terminus by one or more gamma-secretases constitutes the beta-amyloidogenic pathway, i.e. the pathway by which A beta is formed. Cleavage of APP by alpha-secretase produces alpha-sAPP, a secreted form of APP that does not result in beta-amyloid plaque formation. This alternate pathway precludes the formation of A beta peptide. A description of the proteolytic processing fragments of APP is found, for example, in U.S. Patent Nos. 5,441,870; 5,721,130; and 5,942,400.

An aspartyl protease has been identified as the enzyme responsible for processing of APP at the beta-secretase cleavage site. The beta-secretase enzyme has been disclosed using varied nomenclature, including BACE, Asp, and Memapsin. See, for example, Sindha et al., 1999, *Nature* 402:537-554 (p501) and published PCT application WO00/17369.

Several lines of evidence indicate that progressive cerebral deposition of beta-amyloid peptide (A beta) plays a seminal role in the pathogenesis of AD and can precede cognitive symptoms by years or decades. See, for example, Selkoe, 1991, *Neuron* 6:487. Release of A beta from neuronal cells grown in culture and the presence of A beta in cerebrospinal fluid (CSF) of both normal individuals and AD patients has been demonstrated. See, for example, Seubert et al., 1992, *Nature* 359:325-327.

It has been proposed that A beta peptide accumulates as a result of APP processing by beta-secretase, thus inhibition of this enzyme's activity is desirable for the treatment of AD. *In vivo* processing of APP at the beta-secretase cleavage site is thought to be a rate-limiting step in A beta production, and is thus a therapeutic target for the treatment of AD. See for example, Sabbagh, M., et al., 1997, *Alz. Dis. Rev.* 3, 1-19.

BACE1 knockout mice fail to produce A beta, and present a normal phenotype. When crossed with transgenic mice that over express APP, the progeny show reduced amounts of A beta in brain extracts as compared with control animals (Luo et al., 2001 *Nature Neuroscience* 4:231-232). This evidence further supports the proposal that inhibition of beta-secretase activity and reduction of A beta in the brain provides a therapeutic method for the treatment of AD and other beta amyloid disorders.

At present there are no effective treatments for halting, preventing, or reversing the progression of Alzheimer's disease. Therefore, there is an urgent need for pharmaceutical agents capable of slowing the progression of Alzheimer's disease and/or preventing it in the first place.

Compounds that are effective inhibitors of beta-secretase, that inhibit beta-secretase-mediated cleavage of APP, that are effective inhibitors of A beta production, and/or are effective to reduce amyloid beta deposits or plaques, are needed for the treatment and prevention of disease characterized by amyloid beta deposits or plaques, such as AD.

U.S. Patent 5,846,978 discloses bicyclo compounds of the formula 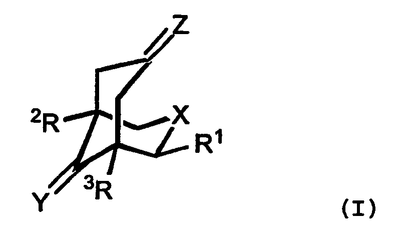 wherein X is --O--, --NH--, --NR⁴-- or --S--;
Y is =O, or forms, with the carbon to which it is attached, 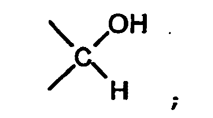 Z is =O, or forms, with the carbon to which it is attached, 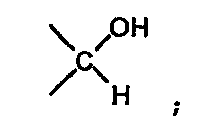 R¹ is
a) H;
b) C₁₋₄ alkyl;
c) C₃₋₇ cycloalkyl;
d) aryl, unsubstituted or substituted one or more times with hydroxy;
e) CH₂R⁵; or
f) 5-7 membered heterocycle; and
R² is
a) C₁₋₄ alkyl;
b) aryl, unsubstituted or substituted with aryl;
c) CH₂R⁶; or
d) heterocycle; and
R³ is
a) CH (OR) R⁷; or
b) CH (NH₂) R⁷; and
R⁴ is
a) C₁₋₄ alkyl;
b) C₃₋₆ cycloalkyl;
c) aryl unsubstituted or substituted with halo or with C₁₋₄ alkyl unsubstituted or substituted one or more times with hydroxy;
d) CH₂R¹; or
e) 5-7 membered heterocycle; and
R⁵ is
a) C₁₋₄ alkyl; or
b) aryl; and
R⁶ is
a) C₁₋₄ alkyl;
b) aryl unsubstituted or substituted with halo or with C₁₋₄ alkyl unsubstituted or substituted one or more times with hydroxy; or
c) 5-7 membered heterocycle; and
R⁷ is
a) H;
b) C₁₋₄ alkyl;
c) aryl unsubstituted or substituted with amino;
d) C₁₋₃ alkylaryl unsubstituted or substituted with amino; or
e) 5-7 membered heterocycle;
or pharmaceutically acceptable salt thereof.

U.S. Patent No. 5,846,978 discloses how to make the above compounds and how to use them as HIV protease inhibitors for the treatment of AIDS.

### SUMMARY OF INVENTION

The present invention relates to methods of treating a patient who has, or in preventing a patient from developing, a disease or condition selected from the group consisting of Alzheimer's disease, for helping prevent or delay the onset of Alzheimer's disease, for helping to slow the progression of Alzheimer's disease, for treating patients with mild cognitive impairment (MCI) and preventing or delaying the onset of Alzheimer's disease in those who would progress from MCI to AD, for treating Down's syndrome, for treating humans who have Hereditary Cerebral Hemorrhage with Amyloidosis of the Dutch-Type, for treating cerebral amyloid angiopathy and preventing its potential consequences, i.e. single and recurrent lobar hemorrhages, for treating other degenerative dementias, including dementias of mixed vascular and degenerative origin, dementia associated with Parkinson's disease, frontotemporal dementias with parkinsonism (FTDP), dementia associated with progressive supranuclear palsy, dementia associated with cortical basal degeneration, or diffuse Lewy body type of Alzheimer's disease and who is in need of such treatment which comprises administration of a therapeutically effective amount of a compound of formula (I) : 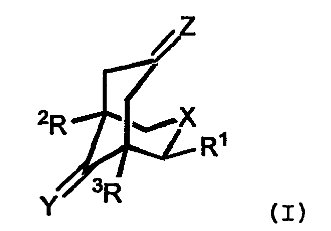 wherein X is --O--, --NH--, --NR⁴-- or --S--;
Y is =O, or forms, with the carbon to which it is attached, 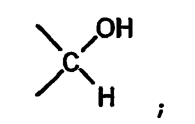 Z is =O, or forms, with the carbon to which it is attached, 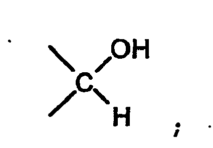 R¹ is
a) H;
b) C₁₋₄ alkyl;
c) C₃₋₇ cycloalkyl;
d) aryl, unsubstituted or substituted one or more times with hydroxy;
e) CH₂R⁵; or
f) 5-7 membered heterocycle; and
R² is
a) C₁₋₄ alkyl;
b) aryl, unsubstituted or substituted with aryl;
c) CH₂R⁶ ; or
d) heterocycle; and
R³ is
a) CH(OH)R⁷; or
b) CH(NH₂)R⁷; and
R⁴ is
a) C₁₋₄ alkyl;
b) C₃₋₆ cycloalkyl;
c) aryl unsubstituted or substituted with halo or with C₁₋₄ alkyl unsubstituted or substituted one or more times with hydroxy;
d) CH₂R¹; or
e) 5-7 membered heterocycle; and
R⁵ is
a) C₁₋₄ alkyl; or
b) aryl; and
R⁶ is
a) C₁₋₄ alkyl;
b) aryl unsubstituted or substituted with halo or with C₁₋₄ alkyl unsubstituted or substituted one or more times with hydroxy; or
c) 5-7 membered heterocycle; and
R⁷ is
a) H;
b) C₁₋₄ alkyl;
c) aryl unsubstituted or substituted with amino;
d) C₁₋₃ alkylaryl unsubstituted or substituted with amino; or
e) 5-7 membered heterocycle;
or pharmaceutically acceptable salt thereof.

### DETAILED DESCRIPTION OF THE INVENTION

In one aspect, the present invention relates to methods of treating a patient who has, or in preventing a patient from developing, a disease or condition selected from the group consisting of Alzheimer's disease, for helping prevent or delay the onset of Alzheimer's disease, for helping to slow the progression of Alzheimer's disease, for treating patients with mild cognitive impairment (MCI) and preventing or delaying the onset of Alzheimer's disease in those who would progress from MCI to AD, for treating Down's syndrome, for treating humans who have Hereditary Cerebral Hemorrhage with Amyloidosis of the Dutch-Type, for treating cerebral amyloid angiopathy and preventing its potential consequences, i.e. single and recurrent lobar hemorrhages, for treating other degenerative dementias, including dementias of mixed vascular and degenerative origin, dementia associated with Parkinson's disease, frontotemporal dementias with parkinsonism (FTDP), dementia associated with progressive supranuclear palsy, dementia associated with cortical basal degeneration, or diffuse Lewy body type of Alzheimer's disease and who is in need of such treatment which comprises administration of a therapeutically effective amount of a compound of formula (I): 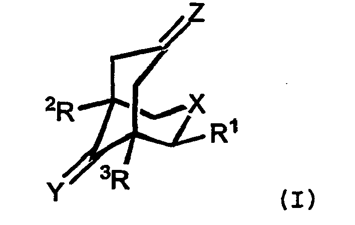 wherein X is --O--, --NH--, --NR⁴-- or --S--;
Y is =O, or forms, with the carbon to which it is attached, 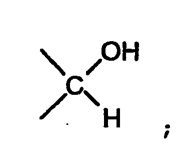 Z is =O, or forms, with the carbon to which it is attached, 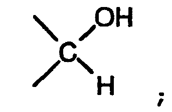 R¹ is
a) H;
b) C₁₋₄ alkyl ;
c) C₃₋₇ cycloalkyl;
d) aryl, unsubstituted or substituted one or more times with hydroxy;
e) CH₂R⁵; or
f) 5-7 membered heterocycle; and
R² is
a) C₁₋₄ alkyl;
b) aryl, unsubstituted or substituted with aryl;
c) CH₂R⁶; or
d) heterocycle; and
R³ is
a) CH(OH)R⁷; or
b) CH (NH₂) R⁷; and
R⁴ is
a) C₁₋₄ alkyl;
b) C₃₋₆ cycloalkyl;
c) aryl unsubstituted or substituted with halo or with C₁₋₄ alkyl unsubstituted or substituted one or more times with hydroxy;
d) CH₂R¹; or
e) 5-7 membered heterocycle; and
R⁵ is
a) C₁₋₄ alkyl; or
b) aryl; and
R⁶ is
a) C₁₋₄ alkyl;
b) aryl unsubstituted or substituted with halo or with C₁₋₄ alkyl unsubstituted or substituted one or more times with hydroxy; or
c) 5-7 membered heterocycle; and
R⁷ is
a) H;
b) C₁₋₄ alkyl;
c) aryl unsubstituted or substituted with amino;
d) C₁₋₃ alkylaryl unsubstituted or substituted with amino; or
e) 5-7 membered heterocycle;
or pharmaceutically acceptable salt thereof.

In a preferred embodiment the methods comprise administration of a compound of the formula II: 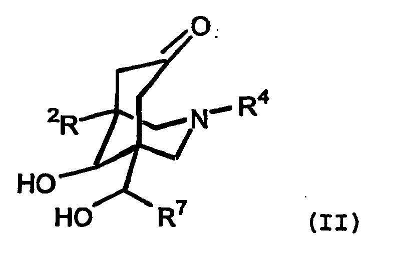 wherein
R² is C₁₋₄ alkylene-aryl; and
R⁴ is C₁₋₄ alkyl, unsubstituted or substituted with aryl, C₃₋₆ cycloalkyl, or 5-7 membered heterocycle;
R⁷ is H, benzyl unsubstituted or substituted with amino;
or pharmaceutically acceptable salt thereof.

In another preferred embodiment the methods comprise administration of compounds that are shown below.
Compound III: 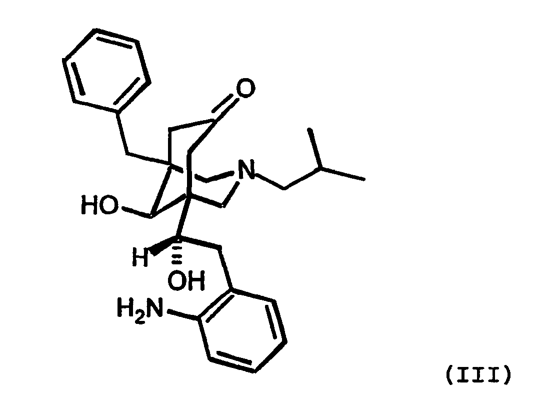 or pharmaceutically acceptable salts thereof; and
Compound IV: 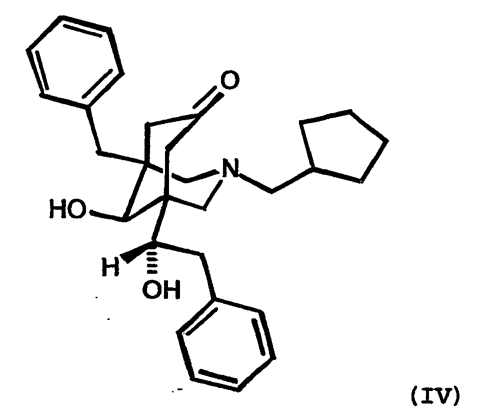 or pharmaceutically acceptable salts thereof.

The compounds useful in the methods of the present invention, may have asymmetric centers and occur as racemates, racemic mixtures and as individual diastereomers, or enantiomers with all isomeric forms being included in the present invention.

When any variable (e.g., aryl, heterocycle, R¹, R², X, Y, or Z, etc.) occurs more than one time in any constituent or in Formula I, its definition on each occurrence is independent of its definition at every other occurrence. Also, combinations of substituents and/or variables are permissible only if such combinations result in stable compounds.

As used herein except where noted, "alkyl" is intended to include both branched- and straight-chain saturated aliphatic hydrocarbon groups having the specified number of carbon atoms (e.g. Me is methyl, Et is ethyl, Pr is propyl, Bu is butyl).

As used herein, with exceptions as noted, "aryl" is intended to mean, for example, phenyl (Ph) or naphthyl.

The term heterocycle or heterocyclic, as used herein except where noted, represents a stable 5- to 7-membered mono- or bicyclic or stable 7- to 10-membered bicyclic heterocyclic ring system, any ring of which may be saturated or unsaturated, and which consists of carbon atoms and from one to three heteroatoms selected from the group consisting of N, O and S, and wherein the nitrogen and sulfur heteroatoms may optionally be oxidized, and the nitrogen heteroatom may optionally be quaternized, and including any bicyclic group in which any of the above-defined heterocyclic rings is fused to a benzene ring. The heterocyclic ring may be attached at any heteroatom or carbon atom which results in the creation of a stable structure . Examples of such heterocyclic elements include piperidinyl, piperazinyl, 2-oxopiperazinyl, 2-oxopiperidinyl, 2-oxopyrrolodinyl, 2-oxoazepinyl, azepinyl, pyrrolyl, 4-piperidonyl, pyrrolidinyl, pyrazolyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, thiadiazoyl, benzopyranyl, benzothiazolyl, benzoxazolyl, furyl, tetrahydrofuryl, tetrahydropyranyl, thienyl, benzothienyl, thiamorpholinyl, thiamorpholinyl sulfoxide, thiamorpholinyl sulfone, and oxadiazolyl.

In one aspect, this method of treatment can be used where the disease is Alzheimer's disease.

In another aspect, this method of treatment can help prevent or delay the onset of Alzheimer's disease.

In another aspect, this method of treatment can help slow the progression of Alzheimer's disease.

In another aspect, this method of treatment can be used where the disease is mild cognitive impairment.

In another aspect, this method of treatment can be used where the disease is Down's syndrome.

In another aspect, this method of treatment can be used where the disease is Hereditary Cerebral Hemorrhage with Amyloidosis of the Dutch-Type.

In another aspect, this method of treatment can be used where the disease is cerebral amyloid angiopathy.

In another aspect, this method of treatment can be used where the disease is degenerative dementias.

In another aspect, this method of treatment can be used where the disease is diffuse Lewy body type of Alzheimer's disease.

In another aspect, this method of treatment can treat an existing disease, such as those listed above.

In another aspect, this method of treatment can prevent a disease, such as those listed above, from developing or progressing.

The methods of the invention employ therapeutically effective amounts: for oral administration from 0.1 mg/day to 1,000 mg/day; for parenteral, sublingual, intranasal, intrathecal administration from 0.5 to 100 mg/day; for depo administration and implants from 0.5 mg/day to 50 mg/day; for topical administration from 0.5 mg/day to 200 mg/day; for rectal administration from 0.5 mg to 500 mg.

In a preferred aspect, the therapeutically effective amounts for oral administration is from 1 mg/day to 100 mg/day; and for parenteral administration from 5 to 50 mg daily.

In a more preferred aspect, the therapeutically effective amounts for oral administration is from 5 mg/day to 50 mg/day.

The present invention includes the use of a compound of formula (I), or a pharmaceutically acceptable salt thereof for the manufacture of a medicament for use in treating a patient who has, or in preventing a patient from developing, a disease or condition selected from the group consisting of Alzheimer's disease, for helping prevent or delay the onset of Alzheimer's disease, for treating patients with mild cognitive impairment (MCI) and preventing or delaying the onset of Alzheimer's disease in those who would progress from MCI to AD, for treating Down's syndrome, for treating humans who have Hereditary Cerebral Hemorrhage with Amyloidosis of the Dutch-Type, for treating cerebral amyloid angiopathy and preventing its potential consequences, i.e. single and recurrent lobar hemorrhages, for treating other degenerative dementias, including dementias of mixed vascular and degenerative origin, dementia associated with Parkinson's disease, frontotemporal dementias with parkinsonism (FTDP), dementia associated with progressive supranuclear palsy, dementia associated with cortical basal degeneration, diffuse Lewy body type of Alzheimer's disease and who is in need of such treatment.

In one aspect, this use of a compound of formula (I) can be employed where the disease is Alzheimer's disease.

In another aspect, this use of a compound of formula (I) can help prevent or delay the onset of Alzheimer's disease.

In another aspect, this use of a compound of formula (I) can help slow the progression of Alzheimer's disease.

In another aspect, this use of a compound of formula (I) can be employed where the disease is mild cognitive impairment.

In another aspect, this use of a compound of formula (I) can be employed where the disease is Down's syndrome.

In another aspect, this use of a compound of formula (I) can be employed where the disease is Hereditary Cerebral Hemorrhage with Amyloidosis of the Dutch-Type.

In another aspect, this use of a compound of formula (I) can be employed where the disease is cerebral amyloid angiopathy.

In another aspect, this use of a compound of formula (I) can be employed where the disease is degenerative dementias.

In another aspect, this use of a compound of formula (I) can be employed where the disease is diffuse Lewy body type of Alzheimer's disease.

In a preferred aspect, this use of a compound of formula (I) is a pharmaceutically acceptable salt of an acid selected from the group consisting of acids hydrochloric, hydrobromic, hydroiodic, nitric, sulfuric, phosphoric, citric, methanesulfonic, CH₃-(CH₂)ₙ-COOH where n is 0 thru 4, HOOC-(CH₂)ₙ₋COOH where n is as defined above, HOOC-CH=CH-COOH, and phenyl-COOH.

The present invention also includes methods for inhibiting beta-secretase activity, for inhibiting cleavage of amyloid precursor protein (APP), in a reaction mixture, at a site between Met596 and Asp597, numbered for the APP-695 amino acid isotype, or at a corresponding site of an isotype or mutant thereof; for inhibiting production of amyloid beta peptide (A beta) in a cell; for inhibiting the production of beta-amyloid plaque in an animal; and for treating or preventing a disease characterized by beta-amyloid deposits in the brain. These methods each include administration of a therapeutically effective amount of a compound of formula (I), or a pharmaceutically acceptable salt thereof.

The present invention also includes a method for inhibiting beta-secretase activity, including exposing said beta-secretase to an effective inhibitory amount of a compound of formula (I), or a pharmaceutically acceptable salt thereof.

In one aspect, this method includes exposing said beta-secretase to said compound *in vitro*.

In another aspect, this method includes exposing said beta-secretase to said compound in a cell.

In another aspect, this method includes exposing said beta-secretase to said compound in a cell in an animal.

In another aspect, this method includes exposing said beta-secretase to said compound in a human.

The present invention also discloses a method for inhibiting cleavage of amyloid precursor protein (APP), in a reaction mixture, at a site between Met596 and Asp597, numbered for the APP-695 amino acid isotype; or at a corresponding site of an isotype or mutant thereof, including exposing said reaction mixture to an effective inhibitory amount of a compound of formula (I), or a pharmaceutically acceptable salt thereof.

In one aspect, this method employs a cleavage site: between Met652 and Asp653, numbered for the APP-751 isotype; between Met 671 and Asp 672, numbered for the APP-770 isotype; between Leu596 and Asp597 of the APP-695 Swedish Mutation; between Leu652 and Asp653 of the APP-751 Swedish Mutation; or between Leu671 and Asp672 of the APP-770 Swedish Mutation.

In another aspect, this method exposes said reaction mixture *in vitro*.

In another aspect, this method exposes said reaction mixture in a cell.

In another aspect, this method exposes said reaction mixture in an animal cell.

In another aspect, this method exposes said reaction mixture in a human cell.

The present invention also includes a method for inhibiting production of amyloid beta peptide (A beta) in a cell, including administering to said cell an effective inhibitory amount of a compound of formula (I), or a pharmaceutically acceptable salt thereof.

In an embodiment, this method includes administering to an animal.

In an embodiment, this method includes administering to a human.

The present invention also discloses a method for inhibiting the production of beta-amyloid plaque in an animal, including administering to said animal an effective inhibitory amount of a compound of formula (I), or a pharmaceutically acceptable salt thereof.

In one embodiment of this aspect, this method includes administering to a human.

The present invention also discloses a method for treating or preventing a disease characterized by beta-amyloid deposits in the brain including administering to a patient an effective therapeutic amount of a compound of formula (I), or a pharmaceutically acceptable salt thereof.

In one aspect, this method employs a compound at a therapeutic amount in the range of from 0.1 to 1000 mg/day.

In another aspect, this method employs a compound at a therapeutic amount in the range of from 15 to 1500 mg/day.

In another aspect, this method employs a compound at a therapeutic amount in the range of from 1 to 100 mg/day.

In another aspect, this method employs a compound at a therapeutic amount in the range of from 5 to 50 mg/day.

In another aspect, this method can be used where said disease is Alzheimer's disease.

In another aspect, this method can be used where said disease is Mild Cognitive Impairment, Down's Syndrome, or Hereditary Cerebral Hemorrhage with Amyloidosis of the Dutch Type.

The present invention also includes a composition including beta-secretase complexed with a compound of formula (I), or a pharmaceutically acceptable salt thereof.

The present invention also includes a method for producing a beta-secretase complex including exposing beta-secretase to a compound of formula (I), or a pharmaceutically acceptable salt thereof, in a reaction mixture under conditions suitable for the production of said complex.

In an embodiment, this method employs exposing *in vitro*.

In an embodiment, this method employs a reaction mixture that is a cell.

The present invention also includes a component kit including component parts capable of being assembled, in which at least one component part includes a compound of formula (I) enclosed in a container.

In an embodiment, this component kit includes lyophilized compound, and at least one further component part includes a diluent.

The present invention also includes a container kit including a plurality of containers, each container including one or more unit dose of a compound of formula (I), or a pharmaceutically acceptable salt thereof.

In an embodiment, this container kit includes each container adapted for oral delivery and includes a tablet, gel, or capsule.

In an embodiment, this container kit includes each container adapted for parenteral delivery and includes a depot product, syringe, ampoule, or vial.

In an embodiment, this container kit includes each container adapted for topical delivery and includes a patch, medipad, ointment, or cream.

The present invention also includes an agent kit including a compound of formula (I), or a pharmaceutically acceptable salt thereof; and one or more therapeutic agents selected from the group consisting of an antioxidant, an anti-inflammatory, a gamma secretase inhibitor, a neurotrophic agent, an acetyl cholinesterase inhibitor, a statin, an A beta peptide, and an anti-A beta antibody.

The present invention provides compounds, compositions, kits, and methods for inhibiting beta-secretase-mediated cleavage of amyloid precursor protein (APP). More particularly, the compounds, compositions, and methods of the invention are effective to inhibit the production of A beta peptide and to treat or prevent any human or veterinary disease or condition associated with a pathological form of A beta peptide.

The compounds, compositions, and methods of the invention are useful for treating humans who have Alzheimer's Disease (AD), for helping prevent or delay the onset of AD, for treating patients with mild cognitive impairment (MCI), and preventing or delaying the onset of AD in those patients who would otherwise be expected to progress from MCI to AD, for treating Down's syndrome, for treating Hereditary Cerebral Hemorrhage with Amyloidosis of the Dutch Type, for treating cerebral beta-amyloid angiopathy and preventing its potential consequences such as single and recurrent lobar hemorrhages; for treating other degenerative dementias, including dementias of mixed vascular and degenerative origin, for treating dementia associated with Parkinson's disease, frontotemporal dementias with parkinsonism (FTDP), dementia associated with progressive supranuclear palsy, dementia associated with cortical basal degeneration, and diffuse Lewy body type AD.

The compounds of the invention possess beta-secretase inhibitory activity. The inhibitory activities of the compounds of the invention are readily demonstrated, for example, using one or more of the assays described herein or known in the art.

The compounds of formula (I) can form salts when reacted with acids. Pharmaceutically acceptable salts are generally preferred over the corresponding compounds of formula (I) since they frequently produce compounds which are usually more water soluble, stable and/or more crystalline. Pharmaceutically acceptable salts are any salt which retains the activity of the parent compound and does not impart any deleterious or undesirable effect on the subject to whom it is administered and in the context in which it is administered. Pharmaceutically acceptable salts include acid addition salts of both inorganic and organic acids.. The preferred pharmaceutically acceptable salts include salts of the following acids acetic, aspartic, benzenesulfonic, benzoic, bicarbonic, bisulfuric, bitartaric, butyric, calcium edetate, camsylic, carbonic, chlorobenzoic, citric, edetic, edisylic, estolic, esyl, esylic, formic, fumaric, gluceptic, gluconic, glutamic, glycollylarsanilic, hexamic, hexylresorcinoic, hydrabamic, hydrobromic, hydrochloric, hydroiodic, hydroxynaphthoic, isethionic, lactic, lactobionic, maleic, malic, malonic, mandelic, methanesulfonic, methylnitric, methylsulfuric, mucic, muconic, napsylic, nitric, oxalic, p-nitromethanesulfonic, pamoic, pantothenic, phosphoric, moriohydrogen phosphoric, dihydrogen phosphoric, phthalic, polygalactouronic, propionic, salicylic, stearic, succinic, succinic, sulfamic, sulfanilic, sulfonic, sulfuric, tannic, tartaric, teoclic and toluenesulfonic. For other acceptable salts*,* see *Int*. *J. Pharm.,* 33, 201-217 (1986) and *J. Pharm. Sci.,* 66(1), 1, (1977).

The present invention provides kits, and methods for inhibiting beta-secretase enzyme activity and A beta peptide production. Inhibition of beta-secretase enzyme activity halts or reduces the production of A beta from APP and reduces or eliminates the formation of beta-amyloid deposits in the brain.

### Methods of the Invention

The compounds of the invention, and pharmaceutically acceptable salts thereof, are useful for treating humans or animals suffering from a condition characterized by a pathological form of beta-amyloid peptide, such as beta-amyloid plaques, and for helping to prevent or delay the onset of such a condition. For example, the compounds are useful for treating Alzheimer's disease, for helping prevent or delay the onset of Alzheimer's disease, for treating patients with MCI (mild cognitive impairment) and preventing or delaying the onset of Alzheimer's disease in those who would progress from MCI to .AD, for treating Down's syndrome, for treating humans who have Hereditary Cerebral Hemorrhage with Amyloidosis of the Dutch-Type, for treating cerebral amyloid angiopathy and preventing its potential consequences, i.e. single and recurrent lobal hemorrhages, for treating other degenerative dementias, including dementias of mixed vascular and degenerative origin, dementia associated with Parkinson's disease, frontotemporal dementias with parkinsonism (FTDP), dementia associated with progressive supranuclear palsy, dementia associated with cortical basal degeneration, and diffuse Lewy body type Alzheimer's disease. The compounds and compositions of the invention are particularly useful for treating, preventing, or slowing the progression of Alzheimer's disease. When treating or preventing these diseases, the compounds of the invention can either be used individually or in combination, as is best for the patient.

With regard to these diseases, the term "treating" means that compounds of the invention can be used in humans with existing disease. The compounds of the invention will not necessarily cure the patient who has the disease but will delay or slow the progression or prevent further progression of the disease thereby giving the individual a more useful life span.

The term "preventing" means that that if the compounds of the invention are administered to those who do not now have the disease but who would normally develop the disease or be at increased risk for the disease, they will not develop the disease. In addition, "preventing" also includes delaying the development of the disease in an individual who will ultimately develop the disease or would be at risk for the disease due to age, familial history, genetic or chromosomal abnormalities, and/or due to the presence of one or more biological markers for the disease, such as a known genetic mutation of APP or APP cleavage products in brain tissues or fluids. By delaying the onset of the disease, compounds of the invention have prevented the individual from getting the disease during the period in which the individual would normally have gotten the disease or reduce the rate of development of the disease or some of its effects but for the administration of compounds of the invention up to the time the individual ultimately gets the disease. Preventing also includes administration of the compounds of the invention to those individuals thought to be predisposed to the disease.

In a preferred aspect, the compounds of the invention are useful for slowing the progression of disease symptoms.

In another preferred aspect, the compounds of the invention are useful for preventing the further progression of disease symptoms.

In treating or preventing the above diseases, the compounds of the invention are administered in a therapeutically effective amount. The therapeutically effective amount will vary depending on the particular compound used and the route of administration, as is known to those skilled in the art.

In treating a patient displaying any of the diagnosed above conditions a physician may administer a compound of the invention immediately and continue administration indefinitely, as needed. In treating patients who are not diagnosed as having Alzheimer's disease, but who are believed to be at substantial risk for Alzheimer's disease, the physician should preferably start treatment when the patient first experiences early pre-Alzheimer's symptoms such as, memory or cognitive problems associated with aging. In addition, there are some patients who may be determined to be at risk for developing Alzheimer's through the detection of a genetic marker such as APOE4 or other biological indicators that are predictive for Alzheimer's disease. In these situations, even though the patient does not have symptoms of the disease, administration of the compounds of the invention may be started before symptoms appear, and treatment may be continued indefinitely to prevent or delay the onset of the disease.

### Dosage Forms and Amounts

The compounds of the invention can be administered orally, parenterally, (IV, IM, depo-IM, SQ, and depo SQ), sublingually, intranasally (inhalation), intrathecally, topically, or rectally. Dosage forms known to those of skill in the art are suitable for delivery of the compounds of the invention.

Compositions are provided that contain therapeutically effective amounts of the compounds of the invention. The compounds are preferably formulated into suitable pharmaceutical preparations such as tablets, capsules, or elixirs for oral administration or in sterile solutions or suspensions for parenteral administration. Typically the compounds described above are formulated into pharmaceutical compositions using techniques and procedures well known in the art.

About 1 to 500 mg of a compound or mixture of compounds of the invention or a physiologically acceptable salt or ester is compounded with a physiologically acceptable vehicle, carrier, excipient, binder, preservative, stabilizer, flavor, etc., in a unit dosage form as called for by accepted pharmaceutical practice. The amount of active substance in those compositions or preparations is such that a suitable dosage in the range indicated is obtained. The compositions are preferably formulated in a unit dosage form, each dosage containing from about 2 to about 100 mg, more preferably about 10 to about 30 mg of the active ingredient. The term "unit dosage from" refers to physically discrete units suitable as unitary dosages for human subjects and other mammals, each unit containing a predetermined quantity of active material calculated to produce the desired therapeutic effect, in association with a suitable pharmaceutical excipient.

To prepare compositions, one or more compounds of the invention are mixed with a suitable pharmaceutically acceptable carrier. Upon mixing or addition of the compound(s), the resulting mixture may be a solution, suspension, emulsion, or the like. Liposomal suspensions may also be suitable as pharmaceutically acceptable carriers. These may be prepared according to methods known to those skilled in the art. The form of the resulting mixture depends upon a number of factors, including the intended mode of administration and the solubility of the compound in the selected carrier or vehicle. The effective concentration is sufficient for lessening or ameliorating at least one symptom of the disease, disorder, or condition treated and may be empirically determined.

Pharmaceutical carriers or vehicles suitable for administration of the compounds provided herein include any such carriers known to those skilled in the art to be suitable for the particular mode of administration. In addition, the active materials can also be mixed with other active materials that do not impair the desired action, or with materials that supplement the desired action, or have another action. The compounds may be formulated as the sole pharmaceutically active ingredient in the composition or may be combined with other active ingredients.

Where the compounds exhibit insufficient solubility, methods for solubilizing may be used. Such methods are known - and include, but are not limited to, using cosolvents such as dimethyleulfoxide (DMSO), using surfactants such as Tween®, and dissolution in aqueous sodium bicarbonate. Derivatives of the compounds, such as salts or prodrugs may also be used in formulating effective pharmaceutical compositions.

The concentration of the compound is effective-for delivery of an amount upon administration that lessens or ameliorates at least one symptom of the disorder for which the compound is administered. Typically, the compositions are formulated for single dosage administration.

The compounds of the invention may be prepared with carriers that protect them against rapid elimination from the body, such as time-release formulations or coatings. Such carriers include controlled release formulations, such as, but not limited to, microencapsulated delivery systems. The active compound is included in the pharmaceutically acceptable carrier in an amount sufficient to exert a therapeutically useful effect in the absence of undesirable side effects on the patient treated. The therapeutically effective concentration may be determined empirically by testing the compounds in known *in vitro* and *in vivo* model systems for the treated disorder.

The compounds and compositions of the invention can be enclosed in multiple or single dose containers. The enclosed compounds and compositions can be provided in kits, for example, including component parts that can be assembled for use. For example, a compound inhibitor in lyophilized form and a suitable diluent may be provided as separated components for combination prior to use. A kit may include a compound inhibitor and a second therapeutic agent for co-administration. The inhibitor and second therapeutic agent may be provided as separate component parts. A kit may include a plurality of containers, each container holding one or more unit dose of the compound of the invention. The containers are preferably adapted for the desired mode of administration, including, but not limited to tablets, gel capsules, sustained-release capsules, and the like for oral administration; depot products, pre-filled syringes, ampoules, vials, and the like for parenteral administration; and patches, medipads, creams, and the like for topical administration.

The concentration of active compound in the drug composition will depend on absorption, inactivation, and excretion rates of the active compound, the dosage schedule, and amount administered as well as other factors known to those of skill in the art.

The active ingredient may be administered at once, or may be divided into a number of smaller doses to be administered at intervals of time. It is understood that the precise dosage and duration of treatment is a function of the disease being treated and may be determined empirically using known testing protocols or by extrapolation from *in vivo* or *in vitro* test data. It is to be noted that concentrations and dosage values may also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed compositions.

If oral administration is desired, the compound should be provided in a composition that protects it from the acidic environment of the stomach. For example, the composition can be formulated in an enteric coating that maintains its integrity in the stomach and releases the active compound in the intestine. The composition may also be formulated in combination with an antacid or other such ingredient.

Oral compositions will generally include an inert diluent or an edible carrier and may be compressed into tablets or enclosed in gelatin capsules. For the purpose of oral therapeutic administration, the active compound or compounds can be incorporated with excipients and used in the form of tablets, capsules, or troches. Pharmaceutically compatible binding agents and adjuvant materials can be included as part of the composition.

The tablets, pills, capsules, troches, and the like can contain any of the following ingredients or compounds of a similar nature: a binder such as, but not limited to, gum tragacanth, acacia, corn starch, or gelatin; an excipient such as microcrystalline cellulose, starch, or lactose; a disintegrating agent such as, but not limited to, alginic acid and corn starch; a lubricant such as, but not limited to, magnesium stearate; a gildant, such as, but not limited to, colloidal silicon dioxide; a sweetening agent such as sucrose or saccharin; and a flavoring agent such as peppermint, methyl salicylate, or fruit flavoring.

When the dosage unit form is a capsule, it can contain, in addition to material of the above type, a liquid carrier such as a fatty oil. In addition, dosage unit forms can contain various other materials, which modify the physical form of the dosage unit, for example, coatings of sugar and other enteric agents. The compounds can also be administered as a component of an elixir, suspension, syrup, wafer, chewing gum or the like. A syrup may contain, in addition to the active compounds, sucrose as a sweetening agent and certain preservatives, dyes and colorings, and flavors.

The active materials can also be mixed with other active materials that do not impair the desired action, or with materials that supplement the desired action.

Solutions or suspensions used for-parenteral, intradermal, subcutaneous, or topical application can include any of the following components: a sterile diluent such as water for injection, saline solution, fixed oil, a naturally occurring vegetable oil such as sesame oil, coconut oil, peanut oil, cottonseed oil, and the like, or a synthetic fatty vehicle such as ethyl oleate, and the like, polyethylene glycol, glycerine, propylene glycol, or other synthetic solvent; antimicrobial agents such as benzyl alcohol and methyl parabens; antioxidants such as ascorbic acid and sodium bisulfite; chelating agents such as ethylenediaminetetraacetic acid (EDTA); buffers such as acetates, citrates, and phosphates; and agents for the adjustment of tonicity such as sodium chloride and dextrose. Parenteral preparations can be enclosed in ampoules, disposable syringes, or multiple dose vials made of glass, plastic, or other suitable material. Buffers, preservatives, antioxidants, and the like can be incorporated as required.

Where administered intravenously, suitable carriers include physiological saline, phosphate buffered saline (PBS), and solutions containing thickening and solubilizing agents such as glucose, polyethylene glycol, polypropyleneglycol, and mixtures thereof. Liposomal suspensions including tissue-targeted liposomes may also be suitable as pharmaceutically acceptable carriers. These may be prepared according to methods known for example, as described in U.S. Patent No. 4,522,811.

The active compounds may be prepared with carriers that protect the compound against rapid elimination from the body, such as time-release formulations or coatings. Such carriers include controlled release formulations, such as, but not limited to, implants and microencapsulated delivery systems, and biodegradable, biocompatible polymers such as collagen, ethylene vinyl acetate, polyanhydrides, polyglycolic acid, polyorthoesters, polylactic acid, and the like. Methods for preparation of such formulations are known to those skilled in the art.

The compounds of the invention can be administered orally, parenterally (IV, IM, depo-IM, SQ, and depo-SQ), sublingually, intranasally (inhalation), intrathecally, topically, or rectally. Dosage forms known to those skilled in the art are suitable for delivery of the compounds of the invention.

Compounds of the invention may be administered enterally or parenterally. When administered orally, compounds of the invention can be administered in usual dosage forms for oral administration as is well known to those skilled in the art. These dosage forms include the usual solid unit dosage forms of tablets and capsules as well as liquid dosage forms such as solutions, suspensions, and elixirs. When the solid dosage forms are used, it is preferred that they be of the sustained release type so that the compounds of the invention need to be administered only once or twice daily.

The oral dosage forms are administered to the patient 1, 2, 3, or 4 times daily. It is preferred that the compounds of the invention be administered either three or fewer times, more preferably once or twice daily. Hence, it is preferred that the compounds of the invention be administered in oral dosage form. It is preferred that whatever oral dosage form is used, that it be designed so as to protect the compounds of the invention from the acidic environment of the stomach. Enteric coated tablets are well known to those skilled in the art. In addition, capsules filled with small spheres each coated to protect from the acidic stomach, are also well known to those skilled in the art.

When administered orally, an administered amount therapeutically effective to inhibit beta-secretase activity, to inhibit A beta production, to inhibit A beta deposition, or to treat or prevent AD is from 0.1 mg/day to 1,000 mg/day. It is preferred that the oral dosage is from 1 mg/day to 100 mg/day. It is more preferred that the oral dosage is from 5 mg/day to 50 mg/day. It is understood that while a patient may be started at one dose, that dose may be varied over time as the patient's condition changes.

Compounds of the invention may also be advantageously delivered in a nano crystal dispersion formulation. Preparation of such formulations is described, for example, in U.S. Patent 5,145,684. Nano crystalline dispersions of HIV protease inhibitors and their method of use are described in U.S. Patent No. 6,045,829. The nano crystalline formulations typically afford greater bioavailability of drug compounds.

The compounds of the invention can be administered parenterally, for example, by IV, IM, depo-IM, SC, or depo-SC. When administered parenterally, a therapeutically effective amount of 0.5 to 100 mg/day, preferably from 5 to 50 mg daily should be delivered. When a depot formulation is used for injection once a month or once every two weeks, the dose should be 0.5 mg/day to 50 mg/day, or a monthly dose of from about 15 mg to about 1,500 mg. In part because of the forgetfulness of the patients with Alzheimer's disease, it is preferred that the parenteral dosage form be a depo formulation.

The compounds of the invention can be administered sublingually. When given sublingually, the compounds of the invention should be given one to four times daily in the amounts described above for IM administration. The compounds of the invention can be administered intranasally. When given by this route, the appropriate dosage forms are a nasal spray or dry powder, as is known to those skilled in the art. The dosage of the compounds of the invention for intranasal administration is the amount described above for IM administration.

The compounds of the invention can be administered intrathecally. When given by this route the appropriate dosage form can be a parenteral dosage form as is known to those skilled in the art. The dosage of the compounds of the invention for intrathecal administration is the amount described above for IM administration.

The compounds of the invention can be administered topically. When given by this route, the appropriate dosage form is a cream, ointment, or patch. Because of the amount of the compounds of the invention to be administered, the patch is preferred. When administered topically, the dosage is from 0.5 mg/day to 200 mg/day. Because the amount that can be delivered by a patch is limited, two or more patches may be used. The number and size of the patch is not important, what is important is that a therapeutically effective amount of the compounds of the invention be delivered as is known to those skilled in the art. The compounds of the invention can be administered rectally by suppository as is known to those skilled in the art. When administered by suppository, the therapeutically effective amount is from 0.5 mg to 500 mg.

The compounds of the invention can be administered by implants as is known to those skilled in the art. When administering a compound of the invention by implant, the therapeutically effective amount is the amount described above for depot administration.

The invention here is the new compounds of the invention and new methods of using the compounds of the invention. Given a particular compound of the invention and a desired dosage form, one skilled in the art would know how to prepare and administer the appropriate dosage form.

The compounds of the invention are used in the same manner, by the same routes of administration, using the same pharmaceutical dosage forms, and at the same dosing schedule as described above, for preventing disease or treating patients with MCI (mild cognitive impairment) and preventing or delaying the onset of Alzheimer's disease in those who would progress from MCI to AD, for treating or preventing Down's syndrome, for treating humans who have Hereditary Cerebral Hemorrhage with Amyloidosis of the Dutch-Type, for treating cerebral amyloid angiopathy and preventing its potential consequences, i.e. single and recurrent lobar hemorrhages, for treating other degenerative dementias, including dementias of mixed vascular and degenerative origin, dementia associated with Parkinson's disease, frontotemporal dementias with parkinsonism (FTDP), dementia associated with progressive supranuclear palsy, dementia associated with cortical basal degeneration, and diffuse Lewy body type of Alzheimer's disease.

The compounds of the invention can be used with each other or with other agents used to treat or prevent the conditions listed above. Such agents include gamma-secretase inhibitors, anti-amyloid vaccines and pharmaceutical agents such as donepezil hydrochloride (ARICEPT Tablets), tacrine hydrochloride (COGNEX Capsules) or other acetylcholine esterase inhibitors and with direct or indirectneurotropic agents of the future.

In addition, the compounds of the invention can also be used with inhibitors of P-glycoproten (P-gp). The use of P-gp inhibitors is known to those skilled in the art. See for example, *Cancer Research,* 53, 4595-4602 (1993), *Clin. Cancer Res.,* 2, 7-12 (1996), *Cancer Research,* 56, 4171-4179 (1996), International Publications WO99/64001 and WO01/10387. The important thing is that the blood level of the P-gp inhibitor be such that it exerts its effect in inhibiting P-gp from decreasing brain blood levels of the compounds of the invention. To that end the P-gp inhibitor and the compounds of the invention can be administered at the same time, by the same or different route of administration, or at different times. The important thing is not the time of administration but having an effective blood level of the P-gp inhibitor.

Suitable P-gp inhibitors include cyclosporin A, verapamil, tamoxifen, quinidine, Vitamin E-TGPS, ritonavir, megestrol acetate, progesterone, rapamycin, 10, 11-methanodibenzosuberane, phenothiazines, acridine derivatives such as GF120918, FK506, VX-710, LY335979, PSC-833, GF-102,918 and other steroids. It is to be understood that additional agents will be found that do the same function and are also considered to be useful.

The P-gp inhibitors can be administered orally, parenterally, (IV, IM, IM-depo, SQ, SQ-depo), topically, sublingually, rectally, intranasally, intrathecally and by implant.

The therapeutically effective amount of the P-gp inhibitors is from 0.1 to 300 mg/kg/day, preferably 0.1 to 150 mg/kg daily. It is understood that while a patient may be started on one dose, that dose may have to be varied over time as the patient's condition changes.

When administered orally, the P-gp inhibitors can be administered in usual dosage forms for oral administration as is known to those skilled in the art. These dosage forms include the usual solid unit dosage forms of tablets and capsules as well as liquid dosage forms such as solutions, suspensions and elixirs. When the solid dosage forms are used, it is preferred that they be of the sustained release type so that the P-gp inhibitors need to be administered only once or twice daily. The oral dosage forms are administered to the patient one thru four times daily. It is preferred that the P-gp inhibitors be administered either three or fewer times a day, more preferably once or twice daily. Hence, it is preferred that the P-gp inhibitors be administered in solid dosage form and further it is preferred that the solid dosage form be a sustained release form which permits once or twice daily dosing. It is preferred that what ever dosage form is used, that it be designed so as to protect the P-gp inhibitors from the acidic environment of the stomach. Enteric coated tablets are well known to those skilled in the art. In addition, capsules filled with small spheres each coated to protect from the acidic stomach, are also well known to those skilled in the art.

In addition, the P-gp inhibitors can be administered parenterally. When administered parenterally they can be administered IV, IM, depo-IM, SQ or depo-SQ. The P-gp inhibitors can be given sublingually. When given sublingually, the P-gp inhibitors should be given one thru four times daily in the same amount as for IM administration.

The P-gp inhibitors can be given intranasally. When given by this route of administration, the appropriate dosage forms are a nasal spray or dry powder as is known to those skilled in the art. The dosage of the P-gp inhibitors for intranasal administration is the same as for IM administration.

The P-gp inhibitors can be given intrathecally. When given by this route of administration the appropriate dosage form can be a parenteral dosage form as is known to those skilled in the art.

The P-gp inhibitors can be given topically. When given by this route of administration, the appropriate dosage form is a cream, ointment or patch. Because of the amount of the P-gp inhibitors needed to be administered the path is preferred. However, the amount that can be delivered by a patch is limited. Therefore, two or more patches may be required. The number and size of the patch is not important, what is important is that a therapeutically effective amount of the P-gp inhibitors be delivered as is known to those skilled in the art. The P-gp inhibitors can be administered rectally by suppository as is known to those skilled in the art.

The P-gp inhibitors can be administered by implants as is known to those skilled in the art.

There is nothing novel about the route of administration nor the dosage forms for administering the P-gp inhibitors. Given a particular P-gp inhibitor, and a desired dosage form, one skilled in the art would know how to prepare the appropriate dosage form for the P-gp inhibitor.

The compounds employed in the methods of the invention can be used in combination, with each other or with other therapeutic agents or approaches used to treat or prevent the conditions listed above. Such agents or approaches include: acetylcholine esterase inhibitors such as tacrine (tetrahydroaminoacridine, marketed as COGNEX® ), donepezil hydrochloride, (marketed as Aricept® and rivastigmine (marketed as Exelon® ); gamma-secretase inhibitors; anti-inflammatory agents such as cyclooxygenase II inhibitors; anti-oxidants such as Vitamin E and ginkolides; immunological approaches, such as, for example, immunization with A beta peptide or administration of anti-A beta peptide antibodies; statins; and direct or indirect neurotropic agents such as Cerebrolysin® , AIT-082 (Emilieu, 2000, Arch. Neurol. 57:454), and other neurotropic agents of the future.

It should be apparent to one skilled in the art that the exact dosage and frequency of administration will depend on the particular compounds employed in the methods of the invention administered, the particular condition being treated, the severity of the condition being treated, the age, weight, general physical condition of the particular patient, and other medication the individual may be taking as is well known to administering physicians who are skilled in this art.

### Inhibition of APP Cleavage

The compounds of the invention inhibit cleavage of APP between Met595 and Asp596 numbered for the APP695 isoform, or a mutant thereof, or at a corresponding site of a different isoform, such as APP751 or APP770, or a mutant thereof (sometimes referred to as the "beta secretase site"). While not wishing to be bound by a particular theory, inhibition of beta-secretase activity is thought to inhibit production of beta amyloid peptide (A beta). Inhibitory activity is demonstrated in one of a variety of inhibition assays, whereby cleavage of an APP substrate in the presence of a beta-secretase enzyme is analyzed in the presence of the inhibitory compound, under conditions normally sufficient to result in cleavage at the beta-secretase cleavage site. Reduction of APP cleavage at the beta-secretase cleavage site compared with an untreated or inactive control is correlated with inhibitory activity. Assay systems that can be used to demonstrate efficacy of the compound inhibitors of the invention are known. Representative assay systems are described, for example, in U.S. Patents No. 5,942,400, 5,744,346, as well as in the Examples below.

The enzymatic activity of beta-secretase and the production of A beta can be analyzed *in vitro* or *in vivo*, using natural, mutated, and/or synthetic APP substrates, natural, mutated, and/or synthetic enzyme, and the test compound. The analysis may involve primary or secondary cells expressing native, mutant, and/or synthetic APP and enzyme, animal models expressing native APP and enzyme, or may utilize transgenic animal models expressing the substrate and enzyme. Detection of enzymatic activity can be by analysis of one or more of the cleavage products, for example, by immunoassay, fluorometric or chromogenic assay, HPLC, or other means of detection. Inhibitory compounds are determined as those having the ability to decrease the amount of beta-secretase cleavage product produced in comparison to a control, where beta-secretase mediated cleavage in the reaction system is observed and measured in the absence of inhibitory compounds.

### Beta-Secretase

Various forms of beta-secretase enzyme are known, and are available and useful for assay of enzyme activity and inhibition of enzyme activity. These include native, recombinant, and synthetic forms of the enzyme. Human beta-secretase is known as Beta Site APP Cleaving Enzyme (BACE), Asp2, and memapsin 2, and has been characterized, for example, in U.S. Patent No. 5,744,346 and published PCT patent applications WO98/22597, WO00/03819, WO01/23533, and WO00/17369, as well as in literature publications (Hussain et al., 1999, *Mol. Cell. Neurosci.* 14:419-427; Vassar et al., 1999, *Science* 286:735-741; Yan et al., 1999, *Nature* 402:533-537; Sinha et al., 1999, *Nature* 40:537-540; and Lin et al., 2000, *PNAS USA* 97:1456-1460). Synthetic forms of the enzyme have also been described (WO98/22597 and WO00/17369). Beta-secretase can be extracted and purified from human brain tissue and can be produced in cells, for example mammalian cells expressing recombinant enzyme.

Preferred methods employ compounds that are effective to inhibit 50% of beta-secretase enzymatic activity at a concentration of less than about 50 micromolar, preferably at a concentration of less than about 10 micromolar, more preferably less than about 1 micromolar, and most preferably less than about 10 nanomolar.

### APP Substrate

Assays that demonstrate inhibition of beta-secretase-mediated cleavage of APP can utilize any of the known forms of APP, including the 695 amino acid "normal" isotype described by Kang et al., 1987, *Nature* 325:733-6, the 770 amino acid isotype described by Kitaguchi et. al., 1981, *Nature* 331:530-532, and variants such as the Swedish Mutation (KM670-1NL) (APP-SW), the London Mutation (V7176F), and others. See, for example, U.S. Patent No. 5,766,846 and also Hardy, 1992, *Nature Genet*. 1:233-234, for a review of known variant mutations. Additional useful substrates include the dibasic amino acid modification, APP-KK disclosed, for example, in WO 00/17369, fragments of APP, and synthetic peptides containing the beta-secretase cleavage site, wild type (WT) or mutated form, e.g., SW, as described, for example, in U.S. Patent No 5,942,400 and WO00/03819.

The APP substrate contains the beta-secretase cleavage site of APP (KM-DA or NL-DA) for example, a complete APP peptide or variant, an APP fragment, a recombinant or synthetic APP, or a fusion peptide. Preferably, the fusion peptide includes the beta-secretase cleavage site fused to a peptide having a moiety useful for enzymatic assay, for example, having isolation and/or detection properties. A useful moiety may be an antigenic epitope for antibody binding, a label or other detection moiety, a binding substrate, and the like.

### Antibodies

Products characteristic of APP cleavage can be measured by immunoassay using various antibodies, as described, for example, in Pirttila et al., 1999, *Neuro. Lett.* 249:21-4, and in U.S. Patent No. 5,612,486. Useful antibodies to detect A beta include, for example, the monoclonal antibody 6E10 (Senetek, St. Louis, MO) that specifically recognizes an epitope on amino acids 1-16 of the A beta peptide; antibodies 162 and 164 (New York State Institute for Basic Research, Staten Island, NY) that are specific for human A beta 1-40 and 1-42, respectively; and antibodies that recognize the junction region of beta-amyloid peptide, the site between residues 16 and 17, as described in U.S. Patent No. 5,593,846.. Antibodies raised against a synthetic peptide of residues 591 to 596 of APP and SW192 antibody raised against 590-596 of the Swedish mutation are also useful in immunoassay of APP and its cleavage products, as described in U.S. Patent Nos. 5,604,102 and 5,721,130.

### Assay Systems

Assays for determining APP cleavage at the beta-secretase cleavage site are well known in the art. Exemplary assays, are described, for example, in U.S. Patent Nos. 5,744,346 and 5,942,400, and described in the Examples below.

### Cell Free Assays

Exemplary assays that can be used to demonstrate the inhibitory activity of the compounds of the invention are described, for example, in WO00/17369, WO 00/03819, and U.S. Patents No. 5,942,400 and 5,744,346. Such assays can be performed in cell-free incubations or in cellular incubations using cells expressing a beta-secretase and an APP substrate having a beta-secretase cleavage site.

An APP substrate containing the beta-secretase cleavage site of APP, for example, a complete APP or variant, an APP fragment, or a recombinant or synthetic APP substrate containing the amino acid sequence: KM-DA or NL-DA, is incubated in the presence of beta-secretase enzyme, a fragment thereof, or a synthetic or recombinant polypeptide variant having beta-secretase activity and effective to cleave the beta-secretase cleavage site of APP, under incubation conditions suitable for the cleavage activity of the enzyme. Suitable substrates optionally include derivatives that may be fusion proteins or peptides that contain the substrate peptide and a modification useful to facilitate the purification or detection of the peptide or its beta-secretase cleavage products. Useful modifications include the insertion of a known antigenic epitope for antibody binding; the linking of a label or detectable moiety, the linking of a binding substrate, and the like.

Suitable incubation conditions for a cell-free *in vitro* assay include, for example: approximately 200 nanomolar to 10 micromolar substrate, approximately 10 to 200 picomolar enzyme, and approximately 0.1 nanomolar to 10 micromolar inhibitor compound, in aqueous solution, at an approximate pH of 4 -7, at approximately 37 degrees C, for a time period of approximately 10 minutes to 3 hours. These incubation conditions are exemplary only, and can be varied as required for the particular assay components and/or desired measurement system. Optimization of the incubation conditions for the particular assay components should account for the specific beta-secretase enzyme used and its pH optimum, any additional enzymes and/or markers that might be used in the assay, and the like. Such optimization is routine and will not require undue experimentation.

One useful assay utilizes a fusion peptide having maltose binding protein (MBP) fused to the C-terminal 125 amino acids of APP-SW. The MBP portion is captured on an assay substrate by anti-MBP capture antibody. Incubation of the captured fusion protein in the presence of beta-secretase results in cleavage of the substrate at the beta-secretase cleavage site. Analysis of the cleavage activity can be, for example, by immunoassay of cleavage products. One such immunoassay detects a unique epitope exposed at the carboxy terminus of the cleaved fusion protein, for example, using the antibody SW192. This assay is described, for example, in U.S. Patent No 5,942,400.

### Cellular Assay

Numerous cell-based assays can be used to analyze beta-secretase activity and/or processing of APP to release A beta. Contact of an APP substrate with a beta-secretase enzyme within the cell and in the presence or absence of a compound inhibitor of the invention can be used to demonstrate beta-secretase inhibitory activity of the compound. Preferably, assay in the presence of a useful inhibitory compound provides at least about 30%, most preferably at least about 50% inhibition of the enzymatic activity, as compared with a non-inhibited control.

In one embodiment, cells that naturally express beta-secretase are used. Alternatively, cells are modified to express a recombinant beta-secretase or synthetic variant enzyme as discussed above. The APP substrate may be added to the culture medium and is preferably expressed in the cells. Cells that naturally express APP, variant or mutant forms of APP, or cells transformed to express an isoform of APP, mutant or variant APP, recombinant or synthetic APP, APP fragment, or synthetic APP peptide or fusion protein containing the beta-secretase APP cleavage site can be used, provided that the expressed APP is permitted to contact the enzyme and enzymatic cleavage activity can be analyzed.

Human cell lines that normally process A beta from APP provide a useful means to assay inhibitory activities of the compounds of the invention. Production and release of A beta and/or other cleavage products into the culture medium can be measured, for example by immunoassay, such as Western blot or enzyme-linked immunoassay (EIA) such as by ELISA.

Cells expressing an APP substrate and an active beta-secretase can be incubated in the presence of a compound inhibitor to demonstrate inhibition of enzymatic activity as compared with a control. Activity of beta-secretase can be measured by analysis of one or more cleavage products of the APP substrate. For example, inhibition of beta-secretase activity against the substrate APP would be expected to decrease release of specific beta-secretase induced APP cleavage products such as A beta.

Although both neural and non-neural cells process and release A beta, levels of endogenous beta-secretase activity are low and often difficult to detect by EIA. The use of cell types known to have enhanced beta-secretase activity, enhanced processing of APP to A beta, and/or enhanced production of A beta are therefore preferred. For example, transfection of cells with the Swedish Mutant form of APP (APP-SW) ; with APP-KK; or with APP-SW-KK provides cells having enhanced beta-secretase activity and producing amounts of A beta that can be readily measured.

In such assays, for example, the cells expressing APP and beta-secretase are incubated in a culture medium under conditions suitable for beta-secretase enzymatic activity at its cleavage site on the APP substrate. On exposure of the cells to the compound inhibitor, the amount of A beta released into the medium and/or the amount of CTF99 fragments of APP in the cell lysates is reduced as compared with the control. The cleavage products of APP can be analyzed, for example, by immune reactions with specific antibodies, as discussed above.

Preferred cells for analysis of beta-secretase activity include primary human neuronal cells, primary transgenic animal neuronal cells where the transgene is APP, and other cells such as those of a stable 293 cell line expressing APP, for example, APP-SW.

### In vivo Assays: Animal Models

Various animal models can be used to analyze beta-secretase activity and /or processing of APP to release A beta, as described above. For example, transgenic animals expressing APP substrate and beta-secretase enzyme can be used to demonstrate inhibitory activity of the compounds of the invention. Certain transgenic animal models have been described, for example, in U.S. Patent Nos.: 5,877,399; 5,612,486; 5,387,742; 5,720,936; 5,850,003; 5,877,015,, and 5,811,633, and in Ganes et al., 1995, Nature 373:523. Preferred are animals that exhibit characteristics associated with the pathophysiology of AD. Administration of the compound inhibitors of the invention to the transgenic mice described herein provides an alternative method for demonstrating the inhibitory activity of the compounds. Administration of the compounds in a pharmaceutically effective carrier and via an administrative route that reaches the target tissue in an appropriate therapeutic amount is also preferred.

Inhibition of beta-secretase mediated cleavage of APP at the beta-secretase cleavage site and of A beta release can be analyzed in these animals by measure of cleavage fragments in the animal's body fluids such as cerebral fluid or tissues. Analysis of brain tissues for A beta deposits or plaques is preferred.

On contacting an APP substrate with a beta-secretase enzyme in the presence of an inhibitory compound of the invention and under conditions sufficient to permit enzymatic mediated cleavage of APP and/or release of A beta from the substrate, the compounds of the invention are effective to reduce beta-secretase-mediated cleavage of APP at the beta-secretase cleavage site and/or effective to reduce released amounts of A beta. Where such contacting is the administration of the inhibitory compounds of the invention to an animal model, for example, as described above, the compounds are effective to reduce A beta deposition in brain tissues of the animal, and to reduce the number and/or size of beta amyloid plaques. Where such administration is to a human subject, the compounds are effective to inhibit or slow the progression of disease characterized by enhanced amounts of A beta, to slow the progression of AD in the, and/or to prevent onset or development of AD in a patient at risk for the disease.

Unless defined otherwise, all scientific and technical terms used herein have the same meaning as commonly understood by one of skill in the art to which this invention belongs. All patents and publications referred to herein are hereby incorporated by reference for all purposes.

### Definitions

Unless defined otherwise, all scientific and technical terms used herein have the same meaning as commonly understood by one of skill in the art to which this invention belongs.

All patents and publications referred to herein are hereby incorporated by reference for all purposes.

APP, amyloid precursor protein, is defined as any APP polypeptide, including APP variants, mutations, and isoforms, for example, as disclosed in U.S. Patent No. 5,766,846.

A beta, amyloid beta peptide, is defined as any peptide resulting from beta-secretase mediated cleavage of APP, including peptides of 39, 40, 41, 42, and 43 amino acids, and extending from the beta-secretase cleavage site to amino acids 39, 40, 41, 42, or 43.

Beta-secretase (BACE1, Asp2, Memapsin 2) is an aspartyl protease that mediates cleavage of APP at the amino-terminal edge of A beta. Human beta-secretase is described, for example, in WO00/17369.

Pharmaceutically acceptable refers to those properties and/or substances that are acceptable to the patient from a pharmacological/toxicological point of view and to the manufacturing pharmaceutical chemist from a physical/chemical point of view regarding composition, formulation, stability, patient acceptance and bioavailability.

A therapeutically effective amount is defined as an amount effective to reduce or lessen at least one symptom of the disease being treated or to reduce or delay onset of one or more clinical markers or symptoms of the disease.

It should be noted that, as used in this specification and the appended claims, the singular forms "a," "an," and "the" include plural referents unless the content clearly dictates otherwise. Thus, for example, reference to a composition containing "a compound" includes a mixture of two or more compounds. It should also be noted that the term "or" is generally employed in its sense including "and/or" unless the content clearly dictates otherwise.

As noted above, depending on whether asymmetric carbon atoms are present, the compounds of the invention can be present as mixtures of isomers, especially as racemates, or in the form of pure isomers, especially optical antipodes.

Salts of compounds having salt-forming groups are especially acid addition salts, salts with bases or, where several salt-forming groups are present, can also be mixed salts or internal salts.

Salts are especially the pharmaceutically acceptable or non-toxic salts of compounds of formula I.

Such salts are formed, for example, by compounds of formula I having an acid group, for example a carboxy group or a sulfo group, and are, for example, salts thereof with suitable bases, such as non-toxic metal salts derived from metals of groups Ia, Ib, IIa and IIb of the Periodic Table of the Elements, for example alkali metal salts, especially lithium, sodium or potassium salts, or alkaline earth metal salts, for example magnesium or calcium salts, also zinc salts or ammonium salts, as well as salts formed with organic amines, such as unsubstituted or hydroxy-substituted mono-, di- or trialkylamines, especially mono-, di- or tri-lower alkylamines, or with quaternary ammonium bases, for example with methyl-, ethyl-, diethyl- or triethyl-amine, mono-, bis- or tris-(2-hydroxy-lower alkyl)-amines, such as ethanol-, diethanol- or triethanolamine, tris(hydroxymethyl)methylamine or 2-hydroxy-tertbutylamine, N,N-di-lower alkyl-N-(hydroxy-lower alkyl)-amines, such as N,N-dimethyl-N-(2-hydroxyethyl)-amine, or N--methyl-D-glucamine, or quaternary ammonium hydroxides, such as tetrabutylammonium hydroxide. The compounds of formula I having a basic group, for example an amino group, can form acid addition salts, for example with suitable inorganic acids, for example hydrohalic acids, such as hydrochloric acid or hydrobromic acid, or sulfuric acid with replacement of one or both protons, phosphoric acid with replacement of one or more protons, e.g. orthophosphoric acid or metaphosphoric acid, or pyrophosphoric acid with replacement of one or more protons, or with organic carboxylic, sulfonic, sulfo or phosphonic acids or N-substituted sulfamic acids, for example acetic acid, propionic acid, glycolic acid, succinic acid, maleic acid, hydroxymaleic acid, methylmaleic acid, fumaric acid, malic acid, tartaric acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid or isonicotinic acid, as well as with amino acids, such as the .alpha.-amino acids mentioned hereinbefore, and with methanesulfonic acid, ethanesulfonic acid, 2-hydroxyethanesulfonic acid, ethane-1,2-disulfonic acid, benzenesulfonic acid, 4-methylbenzenenesulfonic acid, naphthalene-2-sulfonic acid, 2- or 3-phosphoglycerate, glucose-6-phosphate, or N-cyclohexylsulfamic acid (forming cyclamates) or with other acidic organic compounds, such as ascorbic acid. Compounds of formula I having acid and basic groups can also form internal salts.

For isolation and purification purposes it is also possible to use pharmaceutically unacceptable salts.

Preferred pharmaceutically-acceptable salts of the compounds of Formula I (in the form of water- or oil-soluble or dispersible products) comprise those that include the conventional non-toxic salts or the quaternary ammonium salts which are formed, e.g., from inorganic or organic acids or bases. Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others. Other pharmaceutically acceptable salts include the sulfate salt ethanolate and sulfate salts.

### Synthesis of Compounds

Schemes I and II for preparing the compounds useful in the methods of this invention are presented below. Tables I and II which follow the schemes illustrate the compounds that can be synthesized by Schemes I and II, but Schemes I and II are not limited by the compounds in the tables nor by any particular substituents employed in the schemes for illustrative purposes. The examples specifically illustrate the application of the following schemes to specific compounds.

Additional related information on synthetic background is contained in EPO 0337714.

One method for producing Formula I compounds is provided by Scheme I.

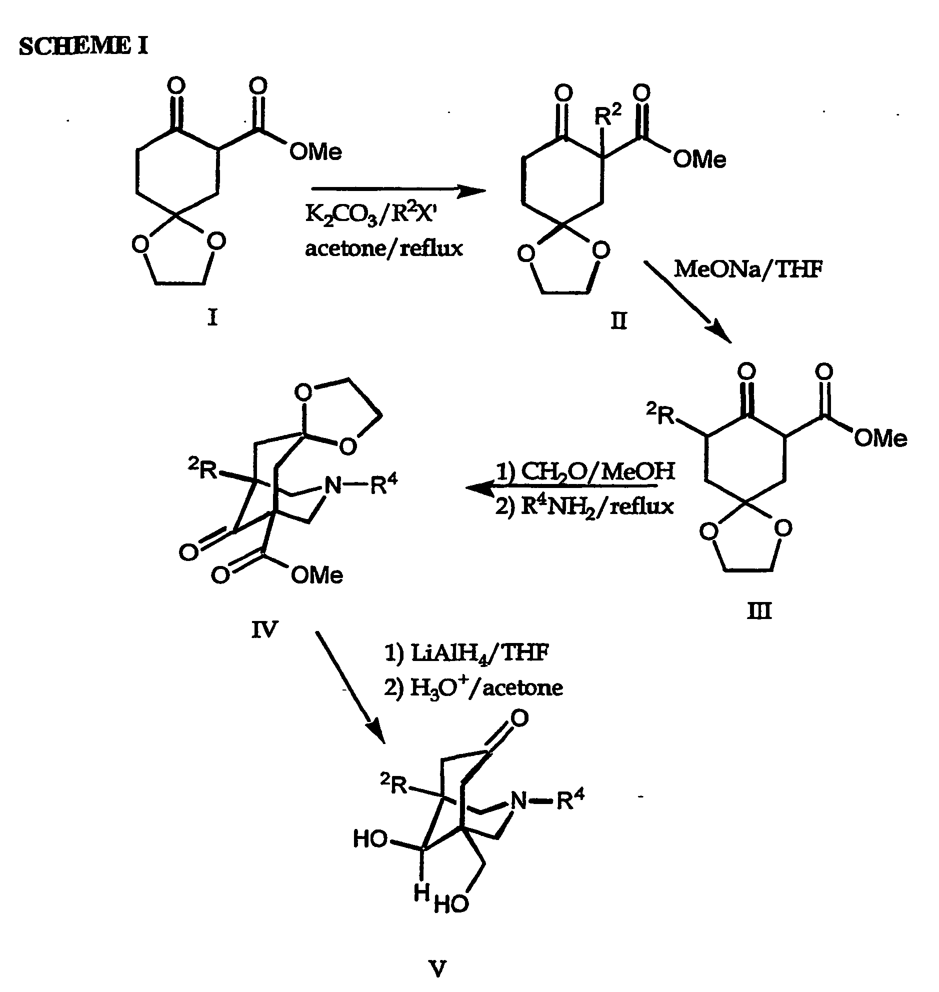

Alkylation of ester I by reaction with R²X' (wherein X' is halo) in base gives II. Reaction with MeONa rearranges II to afford III. Cyclization of R⁴NH₂ gives the azabicyclic (3.3.1) nonane core precursor IV which, after reduction and acid hydrolysis, provides V. Scheme I is illustrated as one embodiment in Example 1.

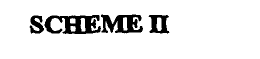

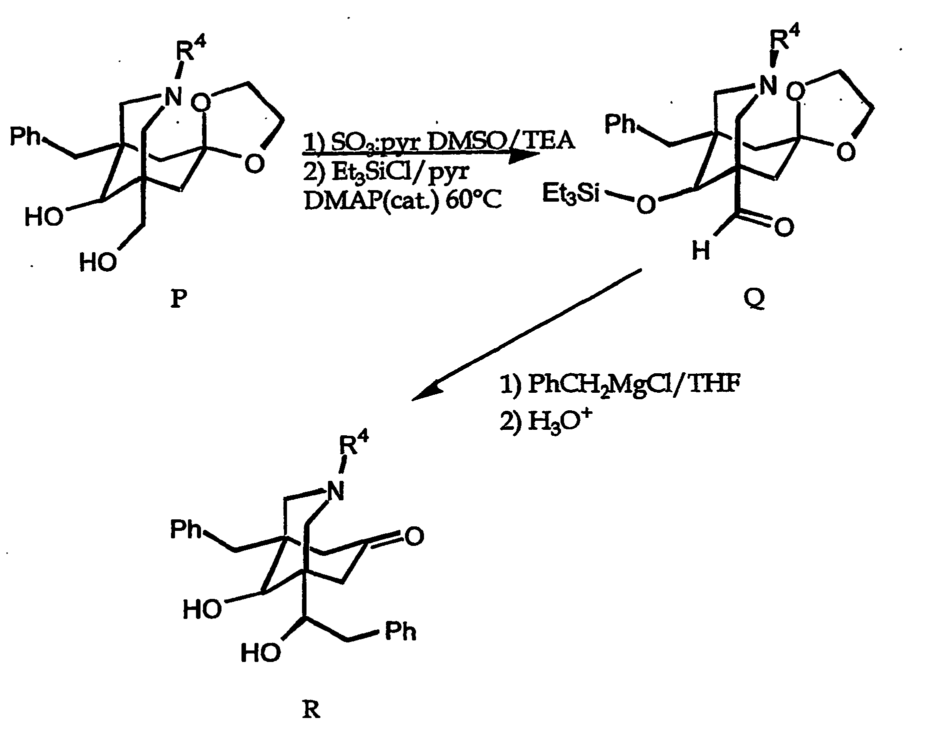

Scheme II outlines another general synthetic method. Alcohol oxidation by treatment of P with SO₃. pyridine complex in DMSO, followed by silylation, gives Q. Alkylation with the appropriate Grignard reagent, followed thereafter with acid treatment, affords R. Scheme II is also illustrated in one embodiment in Example 3.

The compounds useful in the methods of this invention are also illustrated by Tables I-II, which follow.

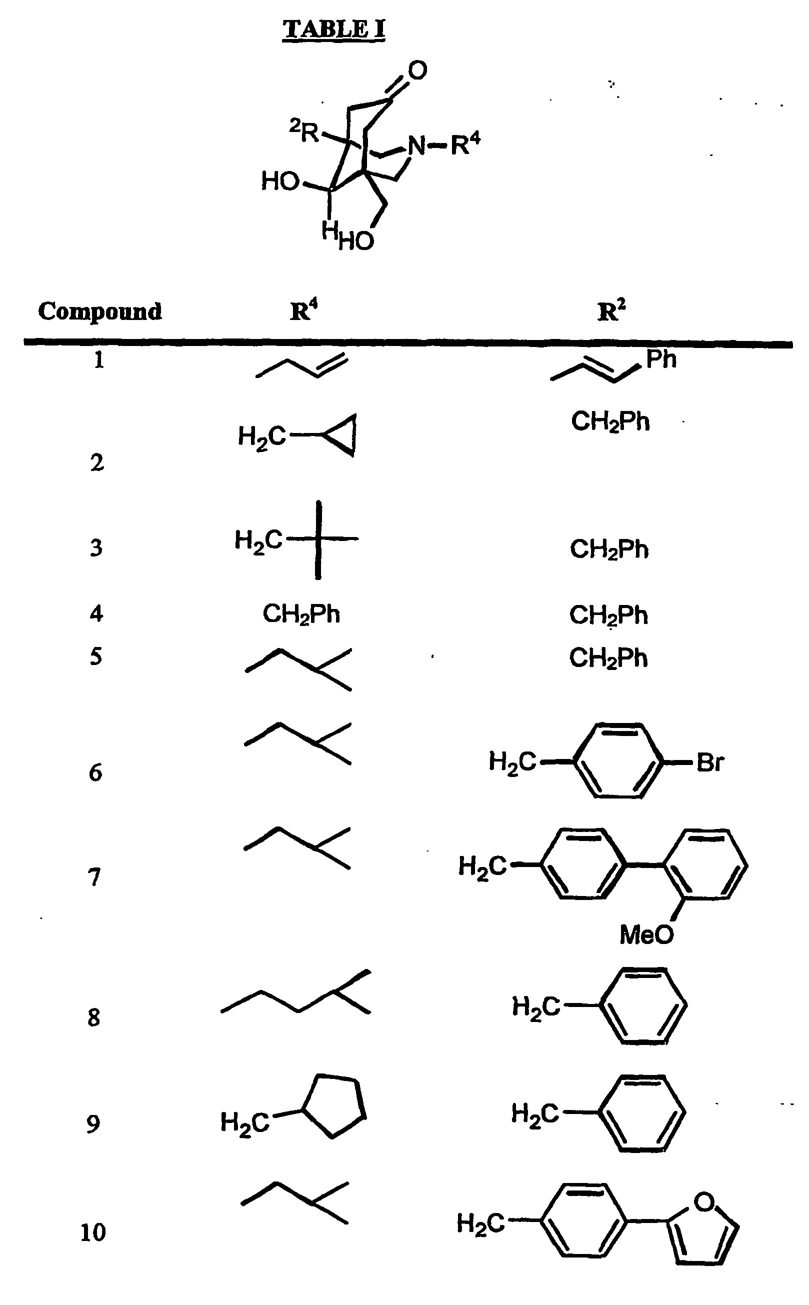

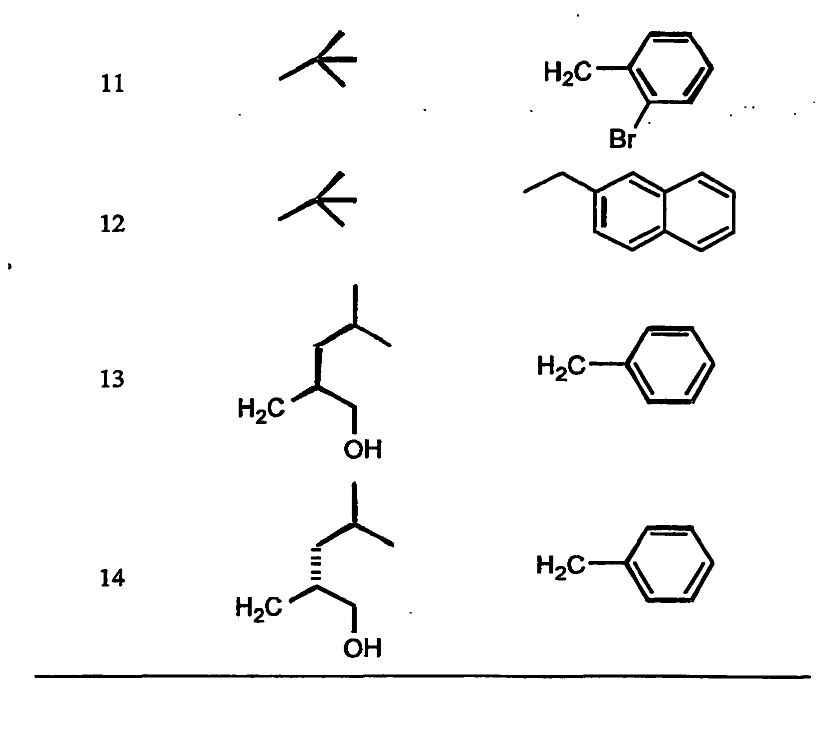

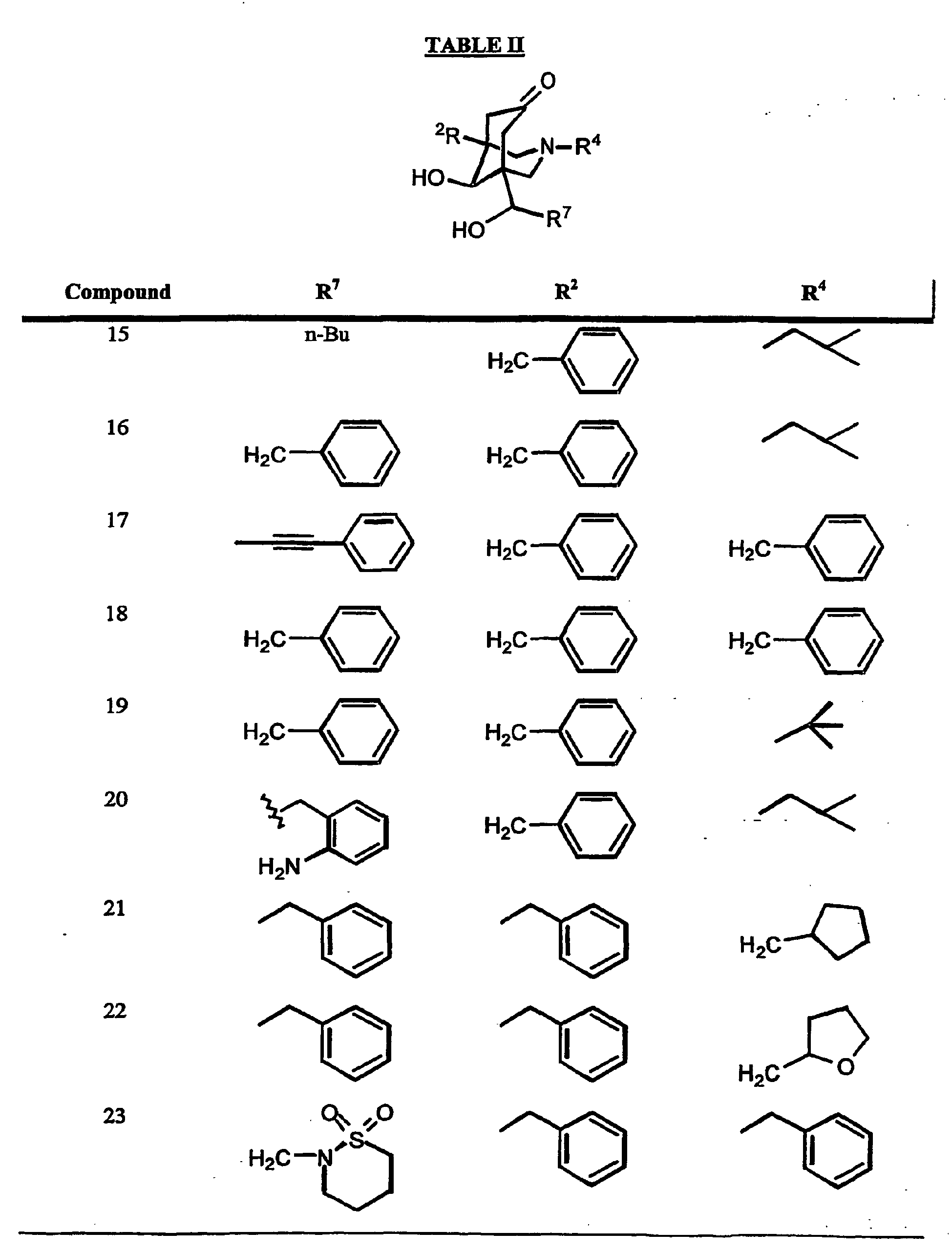

The above synthetic processes are described in detail in U.S. Patent No. 5,846,978, herein incorporated by reference in its entirety.

The present invention may be better understood with reference to the following examples. These examples are intended to be representative of specific embodiments of the invention, and are not intended as limiting the scope of the invention.

### EXAMPLES

### Example A

### Enzyme Inhibition Assay

The compounds of the invention are analyzed for inhibitory activity by use of the MBP-C125 assay. This assay determines the relative inhibition of beta-secretase cleavage of a model APP substrate, MBP-C125SW, by the compounds assayed as compared with an untreated control. A detailed description of the assay parameters can be found, for example, in U.S. Patent No. 5,942,400. Briefly, the substrate is a fusion peptide formed of maltose binding protein (MBP) and the carboxy terminal 125 amino acids of APP-SW, the Swedish mutation. The beta-secretase enzyme is derived from human brain tissue as described in Sinha et al, 1999, *Nature* 40:537-540) or recombinantly produced as the full-length enzyme (amino acids 1-501), and can be prepared, for example, from 293 cells expressing the recombinant cDNA, as described in WO00/47618.

Inhibition of the enzyme is analyzed, for example, by immunoassay of the enzyme's cleavage products. One exemplary ELISA uses an anti-MBP capture antibody that is deposited on precoated and blocked 96-well high binding plates, followed by incubation with diluted enzyme reaction supernatant, incubation with a specific reporter antibody, for example, biotinylated anti-SW192 reporter antibody, and further incubation with streptavidin/alkaline phosphatase. In the assay, cleavage of the intact MBP-C125SW fusion protein results in the generation of a truncated amino-terminal fragment, exposing a new SW-192 antibody-positive epitope at the carboxy terminus. Detection is effected by a fluorescent substrate signal on cleavage by the phosphatase. ELISA only detects cleavage following Leu 596 at the substrate's APP-SW 751 mutation site.

### Specific Assay Procedure:

Compounds are diluted in a 1:1 dilution series to a six-point concentration curve (two wells per concentration) in one 96-plate row per compound tested. Each of the test compounds is prepared in DMSO to make up a 10 millimolar stock solution. The stock solution is serially diluted in DMSO to obtain a final compound concentration of 200 micromolar at the high point of a 6-point dilution curve. Ten (10) microliters of each dilution is added to each of two wells on row C of a corresponding V-bottom plate to which 190 microliters of 52 millimolar NaOAc, 7.9% DMSO, pH 4.5 are pre-added. The NaOAc diluted compound plate is spun down to pellet precipitant and 20 microliters/well is transferred to a corresponding flat-bottom plate to which 30 microliters of ice-cold enzyme-substrate mixture (2.5 microliters MBP-C125SW substrate, 0.03 microliters enzyme and 24.5 microliters ice cold 0.09% TX100 per 30 microliters) is added. The final reaction mixture of 200 micromolar compound at the highest curve point is in 5% DMSO, 20 millimolar NaOAc, 0.06% TX100, at pH 4.5.

Warming the plates to 37 degrees C starts the enzyme reaction. After 90 minutes at 37 degrees C, 200 microliters/well cold specimen diluent is added to stop the reaction and 20 microliters/well was transferred to a corresponding anti-MBP antibody coated ELISA plate for capture, containing 80 microliters/well specimen diluent. This reaction is incubated overnight at 4 degrees C and the ELISA is developed the next day after a 2 hour incubation with anti-192SW antibody, followed by Streptavidin-AP conjugate and fluorescent substrate. The signal is read on a fluorescent plate reader.

Relative compound inhibition potency is determined by calculating the concentration of compound that showed a fifty percent reduction in detected signal (IC₅₀) compared to the enzyme reaction signal in the control wells with no added compound.

### Example B

### Cell Free Inhibition Assay Utilizing a Synthetic APP Substrate

A synthetic APP substrate that can be cleaved by beta-secretase and having N-terminal biotin and made fluorescent by the covalent attachment of Oregon green at the Cys residue is used to assay beta-secretase activity in the presence or absence of the inhibitory compounds of the invention. Useful substrates include the following:

Biotin-SEVNLDAEFRC[Oregon green]KK [SEQ ID NO: 1]

Biotin-SEVKMDAEFRC[Oregon green]KK [SEQ ID NO: 2]

Biotin-GLNIKTEEISEISYEVEFRC[Oregon green]KK [SEQ ID NO: 3]

Biotin-ADRGLTTRPGSGLTNIKTEEISEVNLDAEFC[Oregon green]KK
[SEQ ID NO: 4]

Biotin-FVNQHLCoxGSHLVEALY-LVCoxGERGFFYTPKAC[Oregon green]KK
[SEQ ID NO: 5]

The enzyme (0.1 nanomolar) and test compounds (0.001 - 100 micromolar) are incubated in pre-blocked, low affinity, black plates (384 well) at 37 degrees for 30 minutes. The reaction is initiated by addition of 150 millimolar substrate to a final volume of 30 microliter per well. The final assay conditions are: 0.001 - 100 micromolar compound inhibitor; 0.1 molar sodium acetate (pH 4.5); 150 nanomolar substrate; 0.1 nanomolar soluble beta-secretase; 0.001% Tween 20, and 2% DMSO. The assay mixture is incubated for 3 hours at 37 degrees C, and the reaction is terminated by the addition of a saturating concentration of immunopure streptavidin. After incubation with streptavidin at room temperature for 15 minutes, fluorescence polarization is measured, for example, using a LJL Acqurest (Ex485 nm/ Em530 nm). The activity of the beta-secretase enzyme is detected by changes in the fluorescence polarization that occur when the substrate is cleaved by the enzyme. Incubation in the presence or absence of compound inhibitor demonstrates specific inhibition of beta-secretase enzymatic cleavage of its synthetic APP substrate.

### Example C

### Beta-Secretase Inhibition: P26-P4'SW Assay

Synthetic substrates containing the beta-secretase cleavage site of APP are used to assay beta-secretase activity, using the methods described, for example, in published PCT application **WO00/47618.** The P26-P4'SW substrate is a peptide of the sequence: ' (biotin)CGGADRGLTTRPGSGLTNIKTEEISEVNLDAEF [SEQ ID NO: 6] The P26-P1 standard has the sequence:

(biotin)CGGADRGLTTRPGSGLTNIKTEEISEVNL [SEQ ID NO: 7].

Briefly, the biotin-coupled synthetic substrates are incubated at a concentration of from about 0 to about 200 micromolar in this assay. When testing inhibitory compounds, a substrate concentration of about 1.0 micromolar is preferred. Test compounds diluted in DMSO are added to the reaction mixture, with a final DMSO concentration of 5%. Controls also contain a final DMSO concentration of 5%. The concentration of beta secretase enzyme in the reaction is varied, to give product concentrations with the linear range of the ELISA assay, about 125 to 2000 picomolar, after dilution.

The reaction mixture also includes 20 millimolar sodium acetate, pH 4.5, 0.06% Triton X100, and is incubated at 37 degrees C for about 1 to 3 hours. Samples are then diluted in assay buffer (for example, 145.4 nanomolar sodium chloride, 9.51 millimolar sodium phosphate, 7.7 millimolar sodium azide, 0.05% Triton X405, 6g/liter bovine serum albumin, pH 7.4) to quench the reaction, then diluted further for immunoassay of the cleavage products.

Cleavage products can be assayed by ELISA. Diluted samples and standards are incubated in assay plates coated with capture antibody, for example, SW192, for about 24 hours at 4 degrees C. After washing in TTBS buffer (150 millimolar sodium chloride, 25 millimolar Tris, 0.05% Tween 20, pH 7.5), the samples are incubated with streptavidin-AP according to the manufacturer's instructions. After a one hour incubation at room temperature, the samples are washed in TTBS and incubated with fluorescent substrate solution A (31.2 g/liter 2-amino-2-methyl-1-propanol, 30 mg/liter, pH 9.5). Reaction with streptavidin-alkaline phosphate permits detection by fluorescence. Compounds that are effective inhibitors of beta-secretase activity demonstrate reduced cleavage of the substrate as compared to a control.

### Example D

### Assays using Synthetic Oligopeptide-Substrates

Synthetic oligopeptides are prepared that incorporate the known cleavage site of beta-secretase, and optionally detectable tags, such as fluorescent or chromogenic moieties. Examples of such peptides, as well as their production and detection methods are described in U.S. Patent No: 5,942,400, herein incorporated by reference. Cleavage products can be detected using high performance liquid chromatography, or fluorescent or chromogenic detection methods appropriate to the peptide to be detected, according to methods well known in the art.

By way of example, one such peptide has the sequence (biotin)-SEVNLDAEF [SEQ ID NO: 8], and the cleavage site is between residues 5 and 6. Another preferred substrate has the sequence ADRGLTTRPGSGLTNIKTEEISEVNLDAEF [SEQ ID NO: 9], and the cleavage site is between residues 26 and 27.

These synthetic APP substrates are incubated in the presence of beta-secretase under conditions sufficient to result in beta-secretase mediated cleavage of the substrate. Comparison of the cleavage results in the presence of the compound inhibitor to control results provides a measure of the compound's inhibitory activity.

### Example E

### Inhibition of Beta-Secretase Activity - Cellular Assay

An exemplary assay for the analysis of inhibition of beta-secretase activity utilizes the human embryonic kidney cell line HEKp293 (ATCC Accession No. CRL-1573) transfected with APP751 containing the naturally occurring double mutation Lys651Met52 to Asn651Leu652 (numbered for APP751), commonly called the Swedish mutation and shown to overproduce A beta (Citron et al., 1992, Nature 360:672-674), as described in U.S. Patent No. 5,604,102.

The cells are incubated in the presence/absence of the inhibitory compound (diluted in DMSO) at the desired concentration, generally up to 10 micrograms/ml. At the end of the treatment period, conditioned media is analyzed for beta-secretase activity, for example, by analysis of cleavage fragments. A beta can be analyzed by immunoassay, using specific detection antibodies. The enzymatic activity is measured in the presence and absence of the compound inhibitors to demonstrate specific inhibition of beta-secretase mediated cleavage of APP substrate.

### Example F

### Inhibition of Beta-Secretase in Animal Models of AD

Various animal models can be used to screen for inhibition of beta-secretase activity. Examples of animal models useful in the invention include, but are not limited to, mouse, guinea pig, dog, and the like. The animals used can be wild type, transgenic, or knockout models. In addition, mammalian models can express mutations in APP, such as APP695-SW and the like described herein. Examples of transgenic non-human mammalian models are described in U.S. Patent Nos. 5,604,102, 5,912,410 and 5,811,633.

PDAPP mice, prepared as described in Games et al., 1995, Nature 373:523-527 are useful to analyze in vivo suppression of A beta release in the presence of putative inhibitory compounds. As described in U.S. Patent No. 6,191,166, 4 month old PDAPP mice are administered compound formulated in vehicle, such as corn oil. The mice are dosed with compound (1-30 mg/ml; preferably 1-10 mg/ml). After time, e.g., 3-10 hours, the animals are sacrificed, and brains removed for analysis.

Transgenic animals are administered an amount of the compound inhibitor formulated in a carrier suitable for the chosen mode of administration. Control animals are untreated, treated with vehicle, or treated with an inactive compound. Administration can be acute, i.e., single dose or multiple doses in one day, or can be chronic, i.e., dosing is repeated daily for a period of days. Beginning at time 0, brain tissue or cerebral fluid is obtained from selected animals and analyzed for the presence of APP cleavage peptides, including A beta, for example, by immunoassay using specific antibodies for A beta detection. At the end of the test period, animals are sacrificed and brain tissue or cerebral fluid is analyzed for the presence of A beta and/or beta-amyloid plaques. The tissue is also analyzed for necrosis.

Animals administered the compound inhibitors of the invention are expected to demonstrate reduced A beta in brain tissues or cerebral fluids and reduced beta amyloid plaques in brain tissue, as compared with non-treated controls.

### Example G

### Inhibition of A Beta Production in Human Patients

Patients suffering from Alzheimer's Disease (AD) demonstrate an increased amount of A beta in the brain. AD patients are administered an amount of the compound inhibitor formulated in a carrier suitable for the chosen mode of administration. Administration is repeated daily for the duration of the test period. Beginning on day 0, cognitive and memory tests are performed, for example, once per month.

Patients administered the compound inhibitors are expected to demonstrate slowing or stabilization of disease progression as analyzed by changes in one or more of the following disease parameters: A beta present in CSF or plasma; brain or hippocampal volume; A beta deposits in the brain; amyloid plaque in the brain; and scores for cognitive and memory function, as compared with control, non-treated patients.

### Example H

### Prevention of A Beta Production in Patients at Risk for AD

Patients predisposed or at risk for developing AD are identified either by recognition of a familial inheritance pattern, for example, presence of the Swedish Mutation, and/or by monitoring diagnostic parameters. Patients identified as predisposed or at risk for developing AD are administered an amount of the compound inhibitor formulated in a carrier suitable for the chosen mode of administration. Administration is repeated daily for the duration of the test period. Beginning on day 0, cognitive and memory tests are performed, for example, once per month.

Patients administered the compound inhibitors are expected to demonstrate slowing or stabilization of disease progression as analyzed by changes in one or more of the following disease parameters: A beta present in CSF or plasma; brain or hippocampal volume; amyloid plaque in the brain; and scores for cognitive and memory function, as compared with control, non-treated patients.
All temperatures are in degrees Celsius.

### EXAMPLE 1:

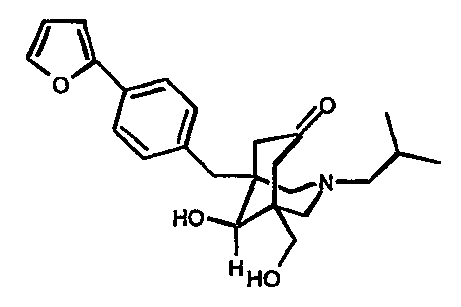

### 5(RS)-((4')-2"-furanyl)methylpheny-9(RS)-hydroxy-1(RS)-hydroxymethyl-3-(2'-methylpropyl-3-azabicyclo[3.3.1] nonan-7-one (Compound 10, Table 1)

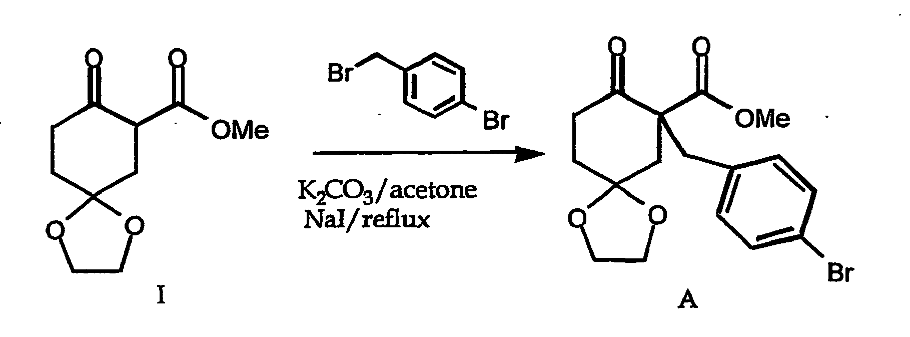

To a solution of 2-carbomethoxy-4-ethylenedioxycyclohexanone I, (3.0 g, 14.0 mmol, Fuchs, P. L. et al., *Syn. Comm.,* 13 (3), 243, 1983) in 100 mL, of acetone was added 4-bromobenzyl bromide (3.67 g, 14.7 mmol), K₂CO₃ (9.69 g, 70.1 mmol) and NaI (210 mg, 1.4 mmol). The heterogenous reaction was heated at reflux for 16 h. The reaction mixture was cooled and filtered through Celite. The filtrate was diluted with 250 mL of Et₂O and the organics were washed with water (2x 20 mL) then brine (50 mL) and dried over MgSO₄. Evaporation of the solvent and flash chromatography (SiO₂; 4:1 Hexane/EtOAc) gave 4.8 g (89%) of A.

¹H NMR (CDCl₃) d 7.40 (d, J=7.8 Hz, 2H), 7.05 (d, J=7.8 Hz, 2H), 3.98 (m, 5H), 3.60 (s, 3H), 3.00 (m, 3H), 2.50 (m, 2H), 1.95 (m, 2H).

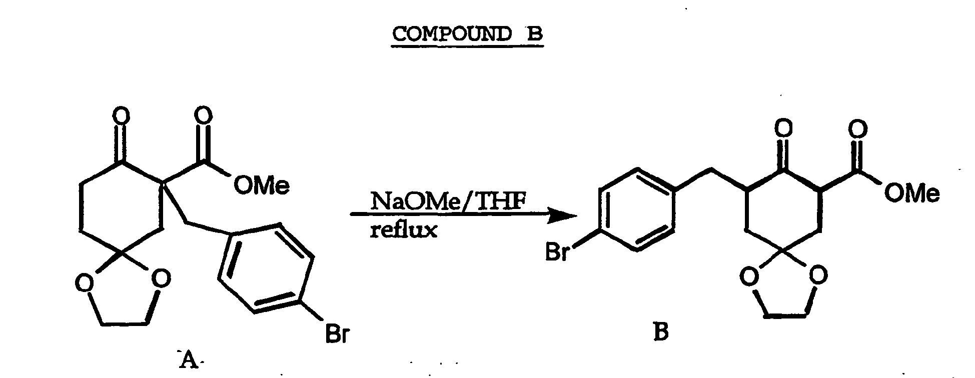

To a slurry of NaH (375 mg, 15.6 mmol) in THF (15 mL) at 0°C. was added MeOH (0.76 mL, 30.7 mmol). After stirring for 5 min, keto ester A (4.8 g, 12.5 mmol) in THF (30 mL) was added dropwise and the solution was warmed to room temperature and stirred for 16 h. The reaction mixture was diluted with 50 mL of EtOAc, then excess NaOMe was quenched with 10 mL of saturated NH₄Cl. The organic phase was separated, washed with brine and dried over MgSO₄. Evaporation of the solvent and flash chromatography (SiO₂; 4:1 Hexane/EtOAc) gave 4.5 g (94%) of B.

¹H NMR (400 MHz, CDCl₃) d 12.6 (s, 1H), 7.40 (d, J=7.8 Hz, 2H), 7.05 (d, J=7.8 Hz, 2H), 3.98 (m, 4H), 3.75 (s, 3H), 3.25 (m, 1H), 2.99 (m, 1H), 2.78 (m, 1H), 2.4-1.8 (m, 4H).

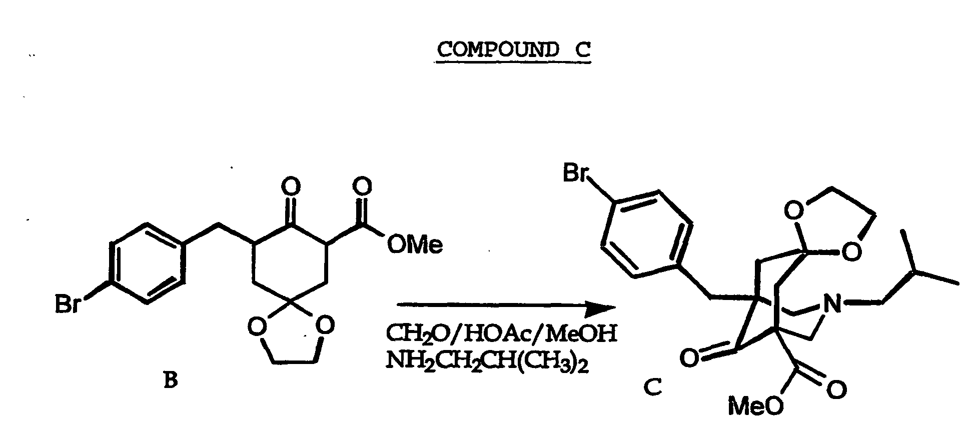

To a solution of keto ester (2.9 g, 7.57 (mmol) B in MeOH (45 mL) and an aqueous solution of formaldehyde (37%, 5.6 mL, 75.7 mmol) was added isobutyl amine (0.9 mL, 9.1 mmol) and HOAc (0.52 mL, 9.1 mmol). The whole was heated at reflux for 16 h. The reaction was cooled to room temperature and the solvent was removed. The residue was dissolved in EtOAc (100 mL) and the resulting solution was washed with sat'd NaHCO₃ (2x 20 mL), water (2x 20 mL) then brine (50 mL) and dried over MgSO₄. Evaporation of the solvent and flash chromatography (SiO₂ gradient; 4:1, 2:1, 1:1 Hexane/EtOAc gave 2.5 g (70%) of C. m.p. 142°-144°C.

¹H NMR (400 MHz, CDCl₃) d 7.40 (d, J=7.8 Hz, 2H), 7.05 (d, J=7.8 Hz, 2H), 3.98 (m, 4H), 3.80 (s, 3H), 3.65 (m, 1H), 3.00 (dd, J=2.5, 11.0 Hz, 1H), 2.85 (d, J=14.1 Hz, 1H), 2.70 (m, 4H), 2.56 (d, J=13.2 Hz, 1H), 2.40 (d, J=13.2 Hz, 1H), 2.20 (m, 3H), 1.70 (m, 1H), 0.90 m, 6H).

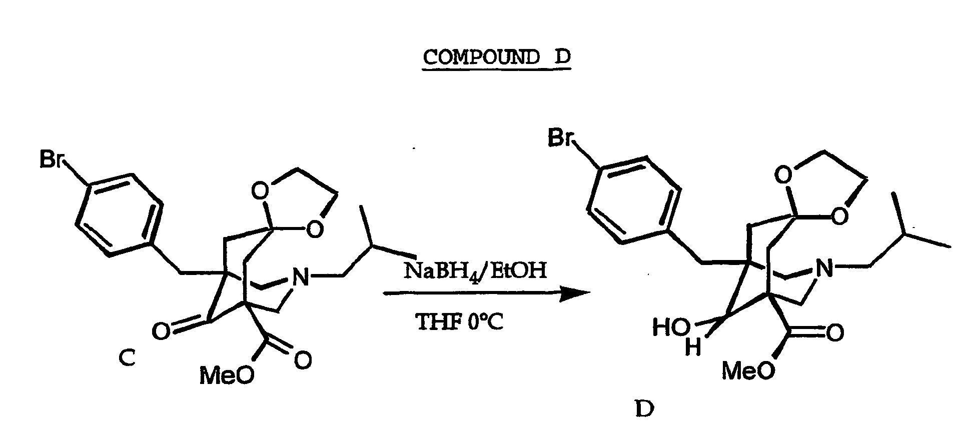

To a solution of ketone (1.8 g, 3.75 mml) C in 18 mL of 1:1:1 EtOH, CH₂Cl₂ and H₂O at 0°C. was added NaBH₄ (142 mg, 3.75 mmol). The solution was stirred for 30 min, then excess NaBH₄ was quenched with 5 mL of acetone. The solution was diluted with EtOAc and washed with water (4x 10 mL) then brine (10 mL). Evaporation of the solvent and column chromatography (SiO₂; 65:35 Hexane/EtOAc) gave 964 mg (53%) of D.

¹H NMR (400 MHz, CDCl₃) d 7.40 (d, J=7.8 Hz, 2H), 7.05 (d, J=7.8 Hz, 2H), 4.50 (d, J=11 Hz, 1H), 4.20-3.90 (m, 4H), 3.75 (s, 3H), 3.40 (d, J=11.2 Hz, 1H), 2.90 (d, J=13.5 Hz, 1H), 2.80 (d, J=12.2 Hz, 1H), 2.60 (m, 2H), 2.40 (d, J=10.6 Hz, 1H), 2.20 (d, J=10.4 Hz, 1H), 2.00-1.80 (m, 5H), 1.70 (m, 1H), 0.90 m, 6H).

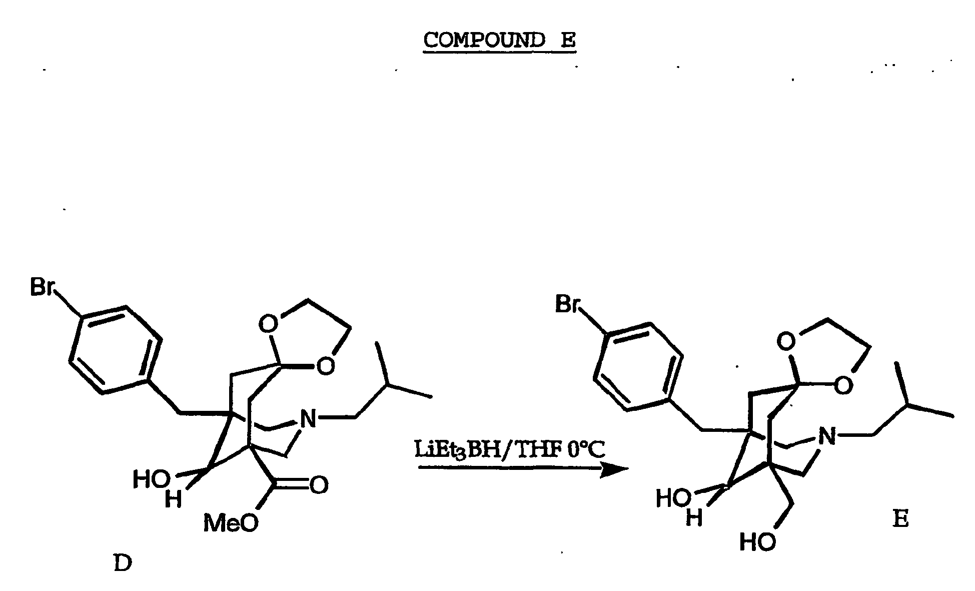

To a solution of ester (964 mg, 2.0 mmol) of D in THF (40 mL) at 0°C. was added LiEt₃BH (6.09 mL, 6.0 mmol). The solution was warmed to room temperature and stirred for 4 hours. Excess LiEt₃ BH was quenched with 5 mL of saturated NaHCO₃. The solution was diluted with Et₂O (50 mL) and washed with saturated NaHCO₃ (3x 10 mL), water (4x 10 mL) and brine (10 mL). Evaporation of the solvent left 800 mg (88%) of crude diol E which was used directly in the next step without purification. m.p.136°-138°C.

¹H NMR (400 MHz, CDCl₃) d 7.39 (d, J=8.2 Hz, 2H), 7.13 (d, J=8.2 Hz, 2H), 4.74 (d, J=11.9 Hz, 1H), 4.05 (m, 4H), 3.57 (d, J=10.8 Hz, 1H), 3.39 (t, J=10.82 Hz, 1H), 3.-6 (d, J=11.9 Hz, 1H), 2.96 (d, J =13.4 Hz, 2H), 2.50 (d, J=13.4 Hz, 1H), 2.34 (d, J=10.6 Hz, 1H), 2.27 (d, J=10.6 Hz, 1H), 2.09 (t, J=11.7 Hz, 2H), 1.95 (d, J=17.3 Hz, 2H), 1. 85 (d, J=14.3 Hz, 1H), 1.77 (m, 1H), 1.63 (t, J=11.1 Hz, 3H), 0.90 (d, J=12.4 Hz, 6H) .

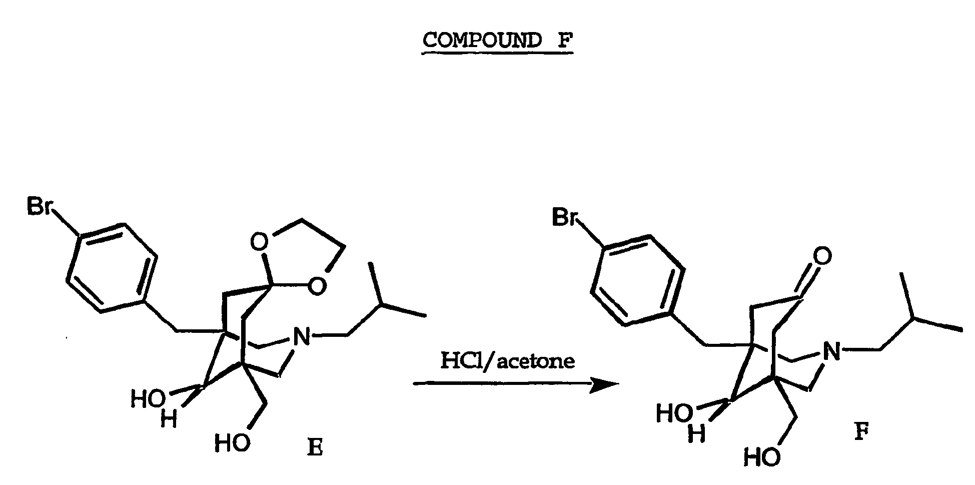

To a solution of ketal (453 mg, 1.0 mmol) E in acetone (8 mL) at 0°C. was added 8 mL of 50% HCl in water. The solution was heated at reflux for 16 h, then cooled to 0°C. Saturated NaHCO₃ solution was added to quench excess HCl. The solution was then washed with EtOAc (3x 10 mL) and the combined organic extracts were dried over MgSO₄. Evaporation of the solvent and trituration of the resulting white solid with Et₂O gave 300 mg (73%) of F. m.p. 155°-156°C.

¹H NMR (400 MHz, CDCl₃) d 7.40 (d, J=7.8 Hz, 2H), 7.05 (d, J=7.8 Hz, 2H), 3.95 (s, 1H), 3.65 (dd, J=4.4, 10.4 Hz, 1H), 3.49 (s, 1H), 3.45 (dd, J=4.6, 10.4 Hz, 1H), 2.80 (m, 2H), 2.60 (d, J=14.2 Hz, 2H), 2.52 (t, J=3.7 Hz, 1H), 2.43 (d, J=11.4 Hz, 1H), 2.35 (d, J=11.1 Hz, 1H), 2.00 (m 4H), 1.83 (d, J=11.2 Hz, 1H), 1.69 (d, J=11.2 Hz, 1H), 1.60 (m, 1H), 0.76 (m, 6H). Anal calc'd for C₂₀ H₂₈ NO₃ Br: C, 58.54;H, 6.88;N, 3.41. Found: C, 58.91;H, 6.88;N, 3.51.

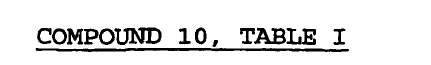

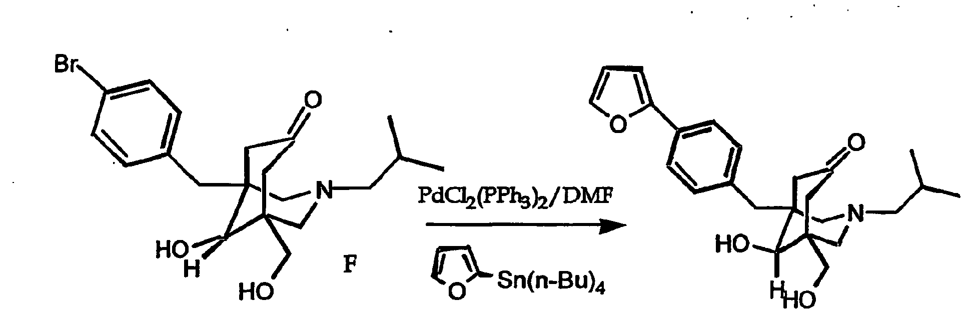

To a solution of the aryl bromide (41 mg, 0.10 mmol) in DMF (0.4 mL) was added 2-(tri-n-butylstannyl) furan (53.5 mg, 0.15 mmol) and PdCl₂ (PPh₃)₂ (1.5 mg, 0.0020 mmol). The resulting yellow-brown solution was stirred at 95°C. for 4 h. The reaction mixture was cooled, diluted with ether and filtered through Celite. The filtrate was washed with water (7x 2 mL), brine (2 mL) and dried over MgSO₄. The yellow oil was subjected to flash chromatography (SiO₂; 95:5:0.5 CHCl₃ /IPA/NH₄ OH) to afford 25 mg (63%) of the title compound as a foam.

¹H NMR (400 MHz, CDCl₃) d 7.60 (d, J=7.8 Hz, 2H), 7.45 (d, J=0.8 Hz, 1H), 7.21 (d, J=7.8 Hz, 2H), 6.62 (d, J=4.2 Hz, 1H), 6.44 (dd, J=4.2, 0.8 Hz, 1H), 3.81 (s, 1H), 3.63 (m, 3H), 3.50 (m, 2H), 2.85 (m, 2H), 2.60 (m, 4H), 2.45 (d, J=14 Hz, 1H), 2.35 (d, J=14 Hz, 1H), 1.8-2.1 (m, 6H), 1.7 (d, J=14 Hz, 1H), 1.6 (m, 2H), 0.77 (d, J=8 Hz, 6H). Anal calc'd for C₂₄ H₃₁ NO₄ . 0.8 H₂O: C, 69.97; H, 7.98; N, 3.40. Found: C, 69.95; H, 7.70; N, 3.52.

### EXAMPLE 2:

5(RS)-methylphenyl-9 (RS) -hydroxy-1(RS)-((1'-hydroxy)-2'-phenyl)-ethyl-3-(2"-methyl)propyl-3-azabicyclo[3.3.1] nonan-7-one (Compound 16), Table II

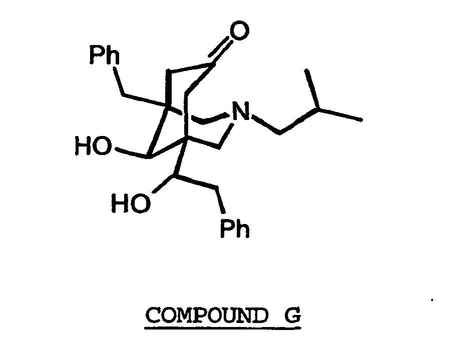

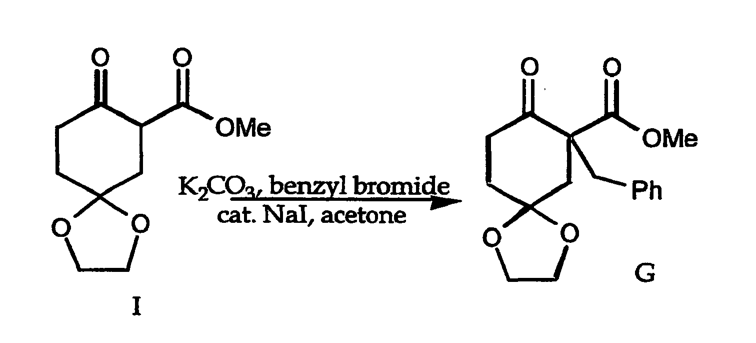

A mixture of I (12.0 g, 56.0 mmnol), benzyl bromide (10.1 g, 7.0 mL, 58.8 mmol), potassium carbonate (48.4 g, 350 mmol), and sodium iodide (250 mg, 1.7 mmol) in acetone (200 mL) was heated at reflux for 16 h. The heterogeneous mixture was then poured into water (150 mL). The aqueous mixture was extracted with ethyl acetate (2x 200 mL). The combined organic layers were dried over Na₂SO₄ and concentrated to give G as a colorless oil (18.7 g). R_{f}=0.16 (20% EtOAc/Hexane)] which was used without further purification.

¹H NMR (400 MHz, CDCl₃) d 7.17-7.26 (m, 5H), 3.91-4.03 (m, 4H), 3.63 (s, 3H), 3.15 (d, 1H, J=13.6 Hz), 3.03 (d, 1H, J=13.6 Hz), 2.97 (ddd, 1H, J=15.0, 8.1, 12.3 Hz), 2.58 (dd, 1H, J=14.1, 2.9 Hz), 2.49 (ddd, 1H, J=15.0, 4.8, 3.7 Hz), 1.91-1.95 (m, 2H), 1.78 (d, 1H, J=13.9 Hz) .

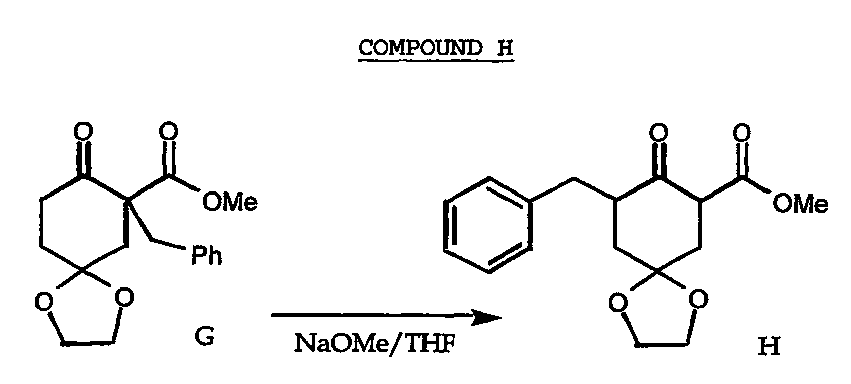

Sodium hydride (2.40 g, 61 mmol, 60 wt % in mineral oil) was washed with hexane to remove mineral oil and then was suspended in tetrahydrofuran (80 mL) at 0°C. Anhydrous methanol (2.15 g, 2.72 mL, 67.1 mmol) was added dropwise over 3 min., followed by warming to 23°C. and stirred for 30 min. The resulting suspension was cooled to 0°C. and G was added via dropping funnel in tetrahydrofuran (40 mL) over 30 min. The reaction mixture was allowed to warm to 23°C. over 1 h and then was stirred at that temperature for 16 h. Aqueous acetic acid (10%, 10 mL) was carefully added and the mixture was poured into saturated NaHCO₃ (100 mL), washed with EtOAc (2x 150 mL), dried (Na₂SO₄) and concentrated to give a brown oil. Recrystallization from MeOH afforded H as white prisms (11.2 g). The mother liquor was concentrated and purified by flash chromatography (20% EtOAc/Hexane) to give a colorless oil which was further purified by recrystallization from Et₂O to give H as white prisms (11.4 g overall, 67% yield for two steps), R_{f}=0.32 (30% EtOAc/Hexane), mp =110°-115°C.

¹H NMR (CDCl₃) d 7.13-7.29 (m, 5H), 3.90-4.00 (m, 4H), 3.81 (dd, 1H, J=13.9, 5.7 Hz), 3.77 (s, 3H), 3.22 (dd, 1H, J=14.1, 5.0 Hz), 2.97-3.05 (m, 1H), 2.45 (dd, 1H, J=14.1, 8.6 Hz), 2.35 (t, 1H, J=13.6 Hz), 2.18 (ddd, 1H, J=13.4, 5.7, 3.8 Hz), 1.98 (ddd, 1H, J=13.2, 5.9, 3.8 Hz), 1.76 (t, 1H, J=13.4 Hz).

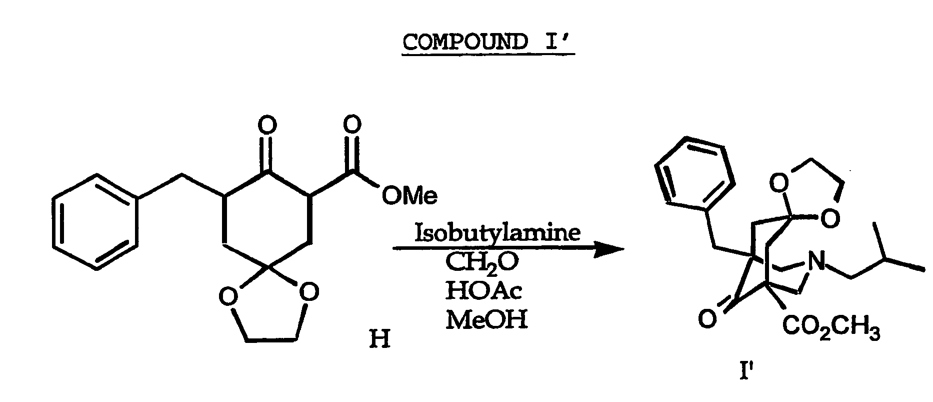

Isobutylamine (4.07 mL, 40.9 mmol), glacial acetic acid (2.28 mL, 39.8 mmol), aqueous formaldehyde (37%, 25.0 mL, 373 mmol), and 3 (10.38 g, 34.1 mmol) were heated at reflux in MeOH (200 mL) for 48 h. The reaction was then concentrated, diluted with EtOAc (125 mL), and poured into saturated NaHCO₃ (100 mL). The biphasic system was partitioned and the aqueous layer was washed with additional EtOAc (2x 100 inL) . The combined organics were dried (Na₂SO₄), concentrated, eluting with Et₂O (50 mL), and then filtered through silica gel washing with Et₂O (500 mL) . The filtrate was concentrated to give 4 as a colorless oil [13.2 g, 96%, R_{f}=0.27 (25% EtOAc/Hexane)]. This material was used without further purification.

¹H NMR (CDCl₃) d 7.15-7.29 (m, 5H), 3.87-4.03 (m, 4H), 3. 80 (s, 3H), 3.02 (dd, 1H, J=11.0, 3.1 Hz), 2.90 (d, 1H, J=13.9 Hz), 2.87 (d, 1H, J=14 .1 Hz), 2. 75 (d, 2H, J=11.0 Hz), 2.68 (dd, 1H, J=13.2, 3.5 Hz), 2.54 (d, 1H, J=13.0 Hz), 2.38 (d, 1H, J=13.2 Hz), 2.14-2.26 (m, 3H), 2.11 (dd, 1H, J=13.2, 3.5), 1.57-1.74 (m, 1H), 0.91 (d, 3H, J=6.6 Hz), 0.89 (d, 3H, J=6.6 Hz).

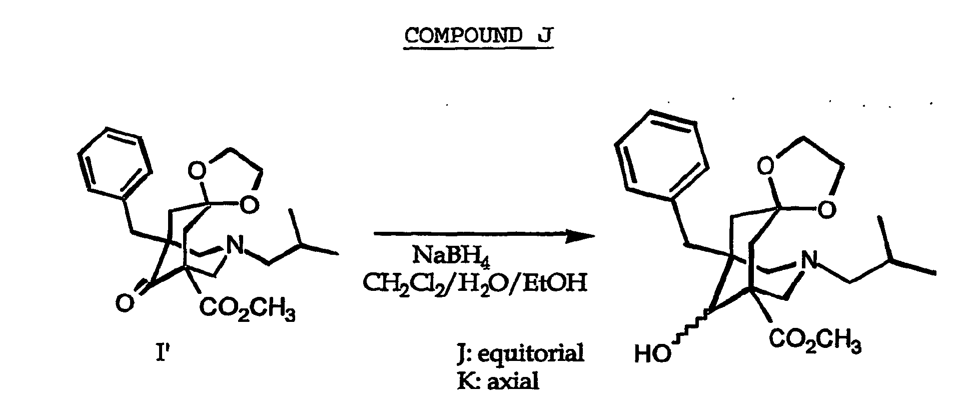

Ethanol was added to a suspension of I' in dichloromethane: water:ethanol (200 mL, 1:2:1) until the solution became homogeneous. Sodium borohydride was added in one gram portions until TLC indicated that I had been consumed (7x 1.0 g). The reaction mixture was cooled to 0°C. and acetone was slowly added until it no longer provoked gas evolution. The resulting mixture was then poured into saturated NaCl (150 mL) and washed with EtOAc (2x 250 mL). The organic layer was dried (Na₂SO₄), concentrated, and purified by flash chromatography (30% EtOAc/Hexane) to give a mixture of J and K as a colorless oil (9.49 g, 72% yield), R_{f}=0.23 (30% EtOAc/Hexane).

Compound J: ¹H NMR (400 MHz, CDCl₃) d 7.22-7.30 (m, 5H), 4.55 (d, 1H, J=11.2 Hz), 3.95-4.15 (m, 4H), 3.73 (s, 3H), 3.47 (d, 1H, J=11.3 Hz), 2.96 (d, 1H, J=13.4 Hz), 2.83 (dd, 1H, J=14.6, 2.1 Hz), 2.68 (d, 1H, J=13.4 Hz), 2.53 (dd, 1H, J=10.6, 2.1 Hz), 2.42 (dd, 1H, J=10.8, 2.2 Hz), 2.19- (d, 1H, J=10.4 Hz), 1.91-2.05 (m, 5H), 1.85 (d, 1H, J=10.7 Hz), 1.58-1.67 (m, 1H), 0.85 (t, 6H, J=7.4 Hz) .

Compound K: ¹H NMR (400 MHz, CDCl₃) d 7.19-7.29 (m, 5H), 4.24 (s, 1H), 3.70-4.08 (m, 4H), 3.70 (s, 3H), 2.89 (d, 1H, J=13.2 Hz), 2.84 (d, 1H, J=10.0 Hz), 2.62 (d, 1H, J=10.8 Hz), 2.49 (d, 1H, J=13.4 Hz), 2.46 (d, 1H, J=11.9 Hz), 2.26 (d, 1H, J=10.8 Hz), 2.20 (d, 1H, J =14.1 Hz), 2.14 (d, 2H, J=7.3 Hz), 1.96 (d, 1H, J=14.5 Hz), 1.90 (d, 1H, J=14.0 Hz), 1.79 (d, 1H, J=14.1 Hz), 1.76-1.81 (m, 1H), 0.89 (d, 3H, J=3.9 Hz), 0.88 (d, 3H, J=4.0 Hz).

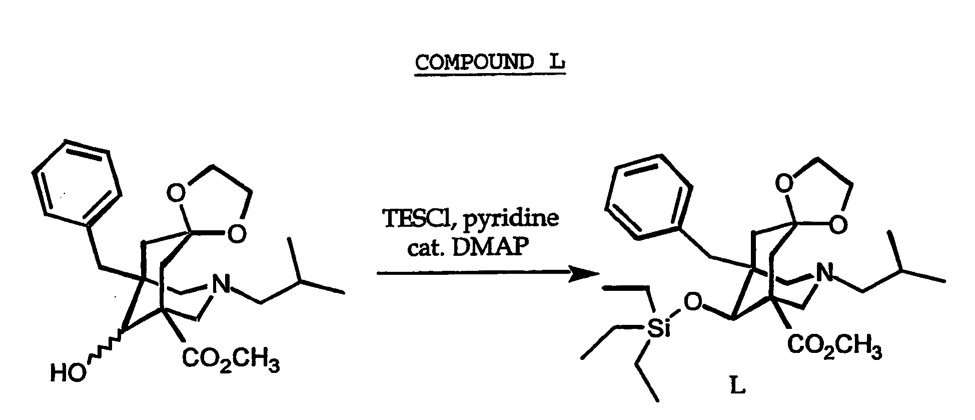

A mixture of J and K (2.16 g, 5.3 mmol, 1:1) in pyridine (40 mL) at 0°C. was treated with chlorotriethylsilane (4.03 g, 4.48 mL, 26.7 mmol). 4-Dimethylaminopyridine (2 mg) was added and the reaction mixture was heated at 60°C. for 4 h. The reaction mixture was then concentrated, and the residue was partitioned between Et₂O (100 mL) and saturated NaHCO₃ (100 mL) . The organic layer was washed with saturated NaCl solution, dried (Na₂SO₄), and concentrated. The resulting oil was purified by flash chromatography (5% EtOAc/Hexane) to give L as a colorless oil (1.14 g, 41% yield of desired isomer), R_{f}=0.33 (10% EtOAc/Hexane).

¹H NMR (400 MHz, CDCl₃) d 7.14-7.31 (m, 5H), 4.04 (s, 1H), 3.76-3.83 (m, 4H), 3.71 (s, 3H), 2.83 (dd, 1H, J=11.3, 1.5 Hz), 2.78 (s, 2H), 2.62 (dd, 1H, J=11.6, 1.5 Hz), 2.40 (dd, 1H, J=11.8, 3.3 Hz), 2.08 (dd, 2H, J=7.3, 1.5 Hz), 2.00 (d, 2H, J=11.6 Hz), 1.88 (d, 1H, J=11.6 Hz), 1.82 (dd, 1H, J=11.5,3.4 Hz), 1.71 (d, 1H, J=11.4 Hz), 1.65-1.70 (m, 1H), 0.97 (t, 9H, J=8.0 Hz), 0.83 (d, 3H, J=7.2 Hz), 0.85 (d, 3H, J=6.7 Hz), 0 . 63 (q, 6H, J=7.9 Hz).

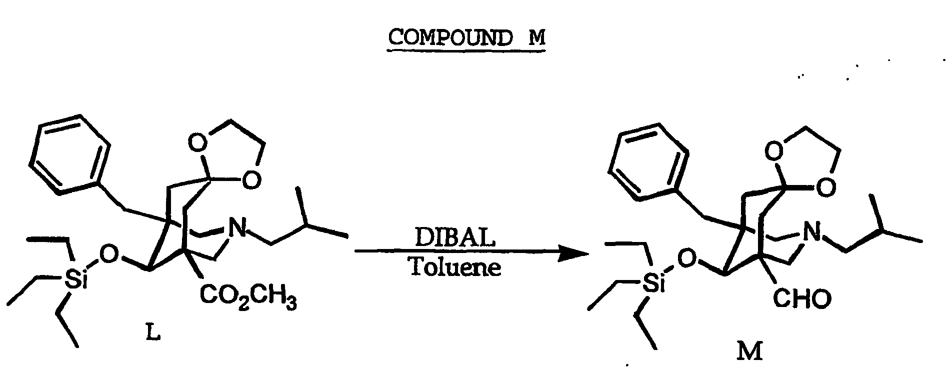

Diisobutylaluminum hydride (1.0M in toluene, 2.91 mL, 2.91 mmol) was cooled to -78°C. and added via cannula to a solution of L (753 mg, 1.45 mmol) in toluene (10 mL) at -78°C. The reaction mixture was stirred for 20 min and then acetone (5 mL) at -78°C. was added via cannula to destroy excess reagent. The mixture was allowed to warm to 23°C., poured into saturated sodium potassium tartrate (100 mL), and the resulting suspension was washed with EtOAc (2x 100 mL). The organic layer was dried (Na₂SO₄) and concentrated to give a mixture of L and M as a colorless oil which was azeotropically dried with toluene (2.times.40 mL) and used without further purification (720 mg, 3:1 mixture of M:L, 75% yield of M), R_{f}=0.41 (50% EtOAc/Hexane)].

¹H NMR (400 MHz, CDCl₃) d 9.66 (s, 1H), 7.13-7.32 (m, 5H), 3.71-3.82 (m, 5H), 2.78 (d, 2H, J=3.4 Hz), 2.74 (dd, 1H, J=11.8, 2.0 Hz), 2.66 (dd, 1H, J=11.8, 2.0 Hz), 2.21 (dd, 1H, J=11.9, 2.6 Hz), 2.10 (dd, 2H, J=7.5, 1.6 Hz), 1.93 (d, 1H, J=1.8 Hz), 1.92 (d, 1H, J=11.9 Hz), 1.83-1.87 (m, 3H), 1.65-1.73 (m, 1H), 0.97 (t, 9H, J=8.0 Hz), 0.85 (d, 3H, J=6.7 Hz), 0.83 (d, 3H, J=7.2 Hz), 0.63 (q, 6H, J=7.9 Hz).

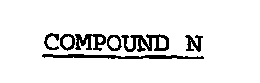

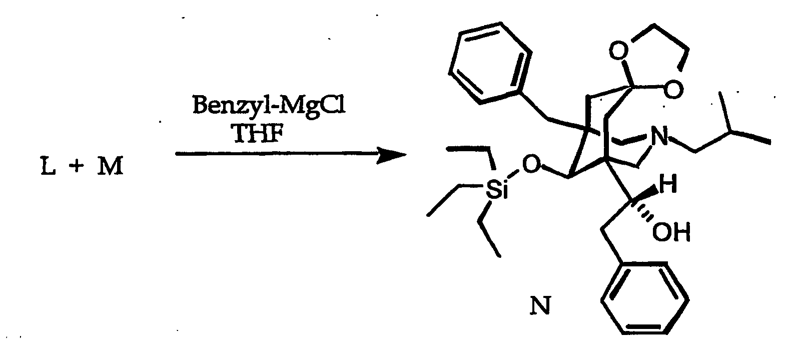

A mixture of M and L (955 mg, 3:1, 1.45 mmol of L) in anhydrous THF (10 mL) at -78°C. was treated with benzylmagnesium chloride (4.75 mL, 9.80 mmol, 2.06M in THF) over 3 min. The reaction mixture was warmed to 0°C. and was kept at that temperature for 1 h. Saturated NH₄Cl was added (5 mL), and the heterogeneous mixture was poured into saturated NaHCO₃ (100 mL) and was washed with EtOAc (2x 100 mL). The organic layer was dried (Na₂SO₄), concentrated and purified by flash chromatography (5% EtOAc/Hexane), to isolate the desired isomer N as a colorless oil (374 mg, 44%). R_{f}=0.23 (10% EtOAc/Hexane).

¹H NMR (400 MHz, CDCl₃) d 7.13-7.35 (m, 10H), 3.74-3.84 (m, 6H), 2.88 (d, 1H, J=13.7 Hz), 2.80 (d, 1H, J=13.4 Hz), 2.77 (d, 1H, J=11.4 Hz), 2.75 (d, 1H, J=13.4 Hz), 2.62 (d, 1H, J=11.4 Hz), 2.47 (dd, 1H, J=13.7, 10.9 Hz), 2.10 (dd, J=7.2, 4.9 Hz), 1.99 (d, 1H, J=11.4 Hz), 1.90 (d, 1H, J=11.5 Hz), 1.85 (d, 1H, J=11.4 Hz), 1.85 (s, 1H), 1.70-1.78 (m, 4H), 0.99 (t, 9H, J=8.0 Hz), 0.86 (d, 3H, J=6.2 Hz), 0.85 (d, 3H, J=6.2 Hz), 0.65 (q, 6H, J=8.0 Hz).

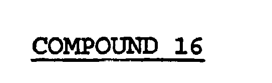

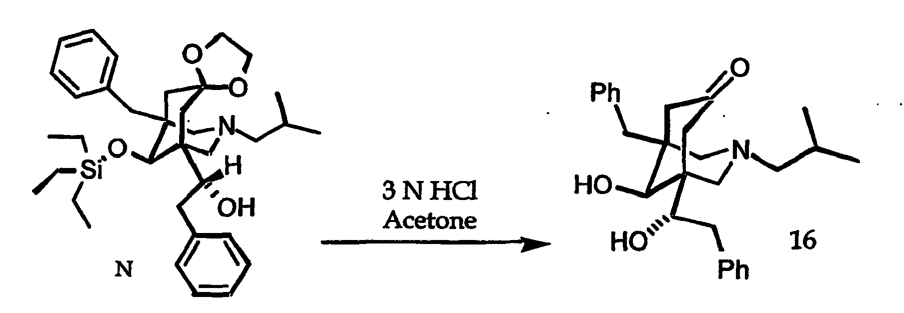

Aqueous HCl (3N, 10 mL) was added to a solution of N (374 mg, 0.64 mmol) in acetone (10 mL). The mixture was heated at 65°C. for 16 h. After cooling to 23°C., the reaction mixture was slowly poured into saturated NaHCO₃ (75 mL). The biphasic system was extracted with EtOAc (2x 150 mL), and the combined organic layers were dried (Na₂SO₄), and concentrated to give 16 as a white solid (265 mg, 99%) . R_{f}=0.13 (30% EtOAc/Hexane), mp=138°-141°C.

¹H NMR (CDCl₃) d 7.16-7.37 (m, 10H), 3.88 (s, 1H), 3.83 (s, 1H), 3.66 (d, 1H, J=11.2 Hz), 2.98 (d, 1H, J=15.9 Hz), 2.90 (d, 1H, J=13.4 Hz), 2.69 (m, 1H), 2.81 (s, 2H), 2.65 (d, 2H, J=13.9 Hz), 2.48 (d, 2H, J=11.4 Hz), 2.00-2.09 (m, 5H), 1.91 (d, 1H, J=11.5 Hz), 1.53-1.66 (m, 1H), 0.79 (d, 3H, J=6.4 Hz), 0.78 (d, 3H, J=6.6 Hz). Anal. Calcd for C₂7 H₃5 NO₃.0.30 H₂ O: C, 75.95; H, 8.40; N, 3.28. Found: C, 75.91; H, 8.30; N, 3.55. HRMS calcd for C₂₇H₃₅NO₃ 422.2695, found 422.2693.

### EXAMPLE 3:

### 5(RS)-methylpheny-9(RS)-hydroxy-1(RS)-((1'-hydroxy)-2'-phenyl)-ethyl-3-benzyl-3-azabicyclo[3.3.1]nonan-7-one (Compound 18), Table II

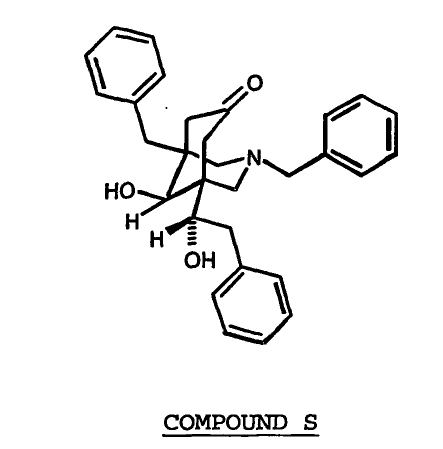

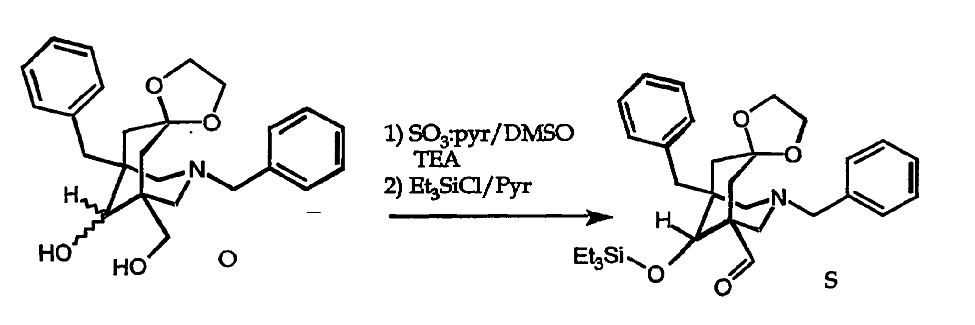

A mixture of diols (162 mg, 0.396 mmol), and SO₃.pyridine complex (189 mg, 1.19 mmol) were dissolved in dry DMSO (4 mL). Triethylamine (0.34 mL) was then added dropwise via syringe. The reaction was allowed to proceed for 1.5 hours at which point it was poured into saturated NH₄Cl solution. The aqueous phase was extracted with EtOAc (2x 50 mL). The organics were combined and washed with H₂O, NaCl and dried (Na₂SO₄) . The extracts were filtered, concentrated to an oil (147 mg) that was used in the next step without purifcation. The crude mixture of hydroxyaldehydes were dissolved in pyridine (6 mL) and treated with triethylsilyl chloride (0.34 mL, 20 mmol) and catalytic amounts of 4-dimethylaminopyridine (10 mg). The reaction was allowed to proceed at 60°C. for 16 hours. The reaction was cooled to room temperature and the volatiles were removed via rotorevaporater. The residue was diluted with Et₂O (75 mL) and washed succesively with NaHCO₃, H₂O and NaCl. The organics were dried over Na₂SO₄ and concentrated. Flash chromatography (9:1, Hexanes/EtOAc) gave the desired compound (S) as an oil (62 mg, 28%).

¹H NMR (400 MHz, CDCl₃) d 9.54 (s, 1H), 7.14-7.31 (m, 10H), 3.82 (m, 5H), 3.54 (AB, JAB=13Hz, 2H), 2.78 (AB, JAB=13.5 Hz, 2H), 2.71 (m, 2H), 2.19 (dd, J=11.7 Hz, 3.1 Hz, 1H), 1.96 (m, 4H), 0.99 (t, J =8 Hz, 9H), 0.65 (q, J=8 Hz, 6H).

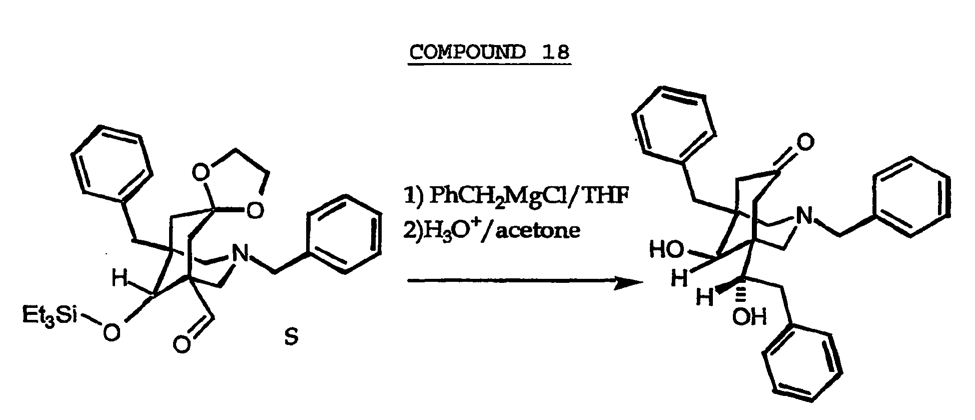

A solution of benzyl magnesium chloride in THF (2.06M, 0.2 mL) was added to a solution of the aldehyde S from step 1 (61 mg, 0.109 mmol) in dry THF (1 mL) at -78°C. The reaction was stirred at -78°C. for 1.5 hours then slowly warmed to room temperature and excess Grignard was quenched with saturated NH₄Cl solution. The reaction was diluted with EtOAc (60 mL) and washed with NH₄Cl, NaCl and dried over Na₂SO₄. The material was immediately hydrolyzed in THF/1N HCl (4:1, 2.5 mL). The desired compound was purified via flash chromatography 1:1 EtOAc/Hexanes and crystallized from Et₂O/Hexanes. m.p. 145.5°-147°C.

¹H NMR (400 MHz, CDCl₃) d 7.17-7.34 (m, 15H), 3.90 (d, J=9.9 Hz, 2H), 3.58 (m, 2H), 3.35 (d, J=13.4 Hz, 1H), 3.00 (d, J=15.7 Hz, 1H), 2.43-2.84 (m, 5H), 2.81 (AB, JAB=13.5 Hz; 2H), 2.0-2.12 (m, 3H). Low resolution FAB Mass spec (M⁺+1)m/z 456. Anal calc'd for C₃₀H₃₃NO₃.0.65 H₂O: C, 77.10; H, 7.40, N, 3.0. Found: C, 71.15; H, 7.24; N, 3.10.

### EXAMPLE 4:

### 5 (RS) -methylpheny-9 (RS) -hydroxy-1 (RS) - ((1'-hydroxy)-2'-(2"'-(tetra-hydro-1,2-thiazine-1,1-dioxide))-ethyl-3-benzyl-3-azabicyclo[3.3.1]nonan-7-one (Compound 23), Table II

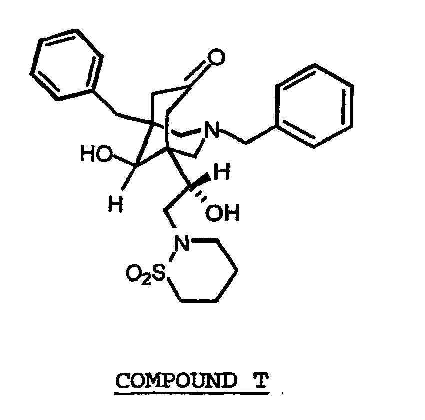

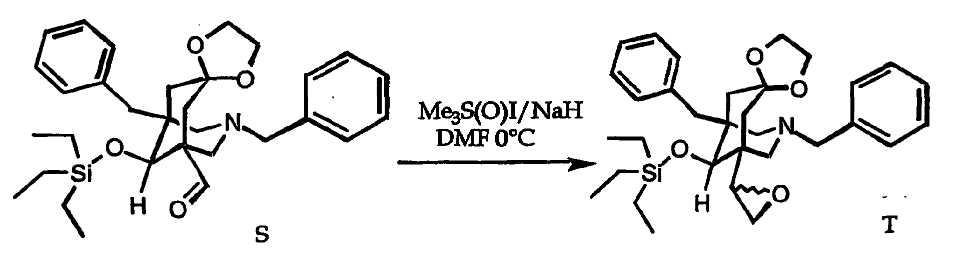

A solution of trimethylsulfoxonium iodide (298 mg, 1.35 mmol), NaH (32 mg, 1.35 mmol) and DMF (4 mL) were stirred at 0°C. for 30 minutes. A solution of aldehyde S from above (152 mg, 0.2709 mmol) in DMF (0.5 mL) was added via syringe. The transfer was completed with two washings of DMF (2x 0.25 mL). The reaction was stirred at 0°C. for 1 hour and quenched with a saturated solution of NH₄Cl. The reaction was poured into NaCl and extracted with Et₂O (3x 35 mL). The organics were combined and washed with H₂O, NaCl, and dried over Na₂SO₄. Flash chromatography using 8:1 Hexane/EtOAc gave 68 mg of one diastereomer and 25 mg of a second diastereomer (both oils).

Major more polar isomer ¹H NMR (400 MHz, CDCl₃), d 7.14-7.3 (m, 10H), 3. 81 (m, 4H), 3. 60 (s, 1H), 3.50 (AB, JAB=13.2 Hz, 2H), 2.67 (br t, J=3 Hz, 1H), 2. 78 (AB, JAB=13.5 Hz, 2H), 2.72 (d, J=11.4 Hz, 1H), 2.66 (m, 2H), 2.60 (d, J=11.4 Hz, 1H), 1.96 (d, J=11.4 Hz, 1H), 1.89 (d, J=11.5 Hz, 1H), 1.82 (dd, J=11.5 Hz, 2.9 Hz, 1H), 1.76 (dd, J=11.4 Hz, 9.4 Hz, 1H), 1.53 (dd, J=11.9 Hz, 3.1 Hz, 1H), 1.00 (t, J=7.5 Hz, 9H), 0.65 (q, J=7.5 Hz, 6H).

Minor less polar isomer ¹H NMR (400 MHz, CDCl₃), d 7.10-7.32 (m, 10H), 3. 79 (m, 4H), 3.50 (AB, JAB=13.2 Hz, 2H), 3.23 (s, 1H), 2.62-2.77 (m, 7H), 1.80-1.96 (m, 4H), 1.70 (dd, J=11.5 Hz, 2.5 Hz, 1H), 0.98 (t, J=7.7 Hz, 9H), 0.64 (q, J=7.7 Hz, 6H).

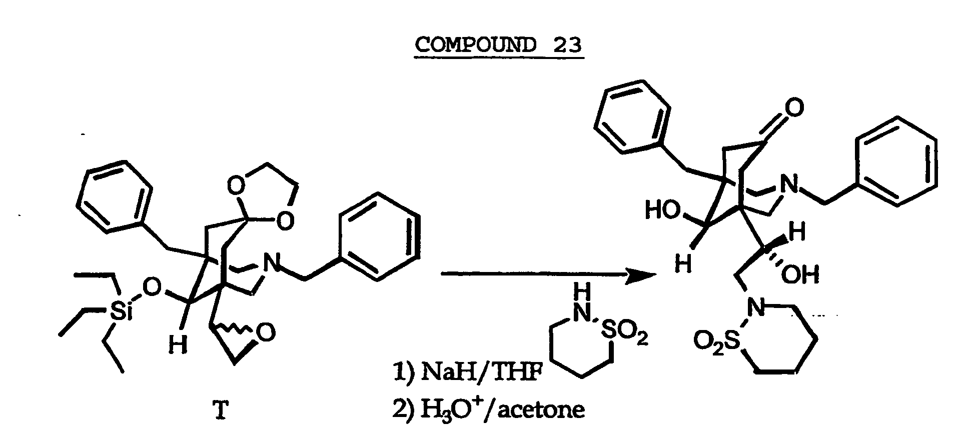

NaH (8 mg, 60 wt % in mineral oil, 200 mmol) was added to a solution of tetrahydro-1,2-thiazine-1,1-dioxide (34 mg, 250 mmol) in N,N-dimethylformamide (1 mL) and the resulting mixture was stirred at 23°C. for 30 min. A solution of T (27 mg, 50 mmol) in N,N-dimethylformamide (1 mL) was added and the mixture was heated at 65°C . for 16 h. The reaction was cooled to room temperature and NH₄Cl solution (2 mL) was added. The reaction mixture was poured into water (50 mL) and the resulting aqueous mixture was extracted with Et₂O (2x 50 mL). The combined organic extracts were washed with water (25 mL), dried (Na₂SO₄), and concentrated to give a mixture of starting material and desired product which was used without further purification.

A solution containing the crude reaction mixture from above (30 mg) in acetone (3 mL) was treated with aqueous hydrochloric acid (3N, 3 mL) and the colorless solution was heated at 65°C. for 12 h. The reaction mixture was cooled to room temperature and slowly poured into saturated NaHCO₃ (25 mL). The resulting suspension was washed with EtOAc (30 mL) and the organic layer was dried (Na₂SO₄), and concentrated to give a crude oil which was purified by flash chromatography (70% EtOAc/Hexane) to give 23 as a white solid (15 mg, 58% for 2 steps). R_{f}=0.13 (70% EtOAc/Hexane), mp=163°-164°C.

¹H NMR (400 MHz, CDCl₃) d 7.13-7.33 (m, 10H), 3.80 (bs, 1H), 3.63 (bd, 1H, J=10.1 Hz), 3.59 (bs, 1H), 3.51 (d, 1H, J=13.4 Hz), 3.21-3.39 (m, 4H), 3.01-3.06 (m, 3H), 2.86 (d, 1H, J=15.6 Hz), 2.79 (d, 1H, J=13.4 Hz), 2.73 (d, 1H, J=13.4 Hz), 2.66 (d, 1H, J=15.6 Hz), 2.53 (dd, 1H, J=11.3, 1.8 Hz), 2.37 (dd, 1H, J=10.9, 1.9 Hz), 2.20 (quintet, 2H, J=6.0 Hz), 1.93-2.09 (m, 4H), .1.64-1.66 (m, 2H). Anal calcd. for C₂₈ H₃₆ N₂SO₅•0.80 H₂O: C, 63.81; H, 7.19; N, 5.31. Found: C, 63.81; H, 7.02; N, 5.18; HRMS calcd for C₂₈ H₃₆ N₂SO₅ 513.2423 , found 513.2437 .

### EXAMPLE 5

### 5(RS)-methylpheny-9(RS)-hydroxy-1(RS)-((1'-hydroxy)-2'-(2"-amino)-phenyl)-ethyl-3-benzyl-3-azabicyclo [3.3.1] nonan-7-one (Compound 20), Table II

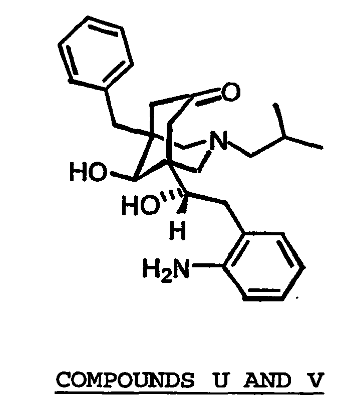

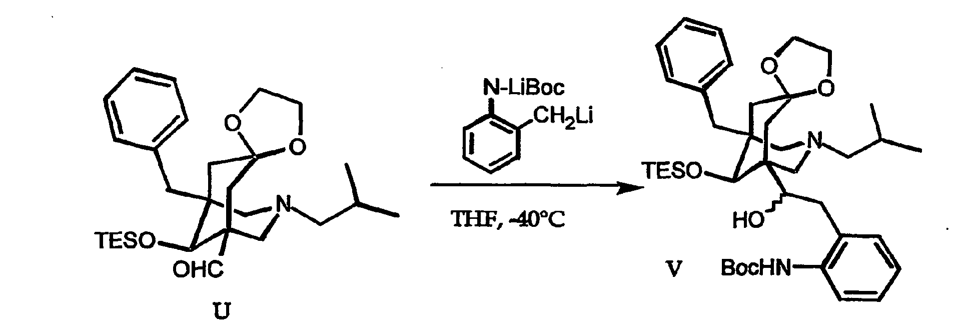

A solution of tert-butyllithium in pentane (1.7M, 3.00 mL, 5.10 mmol, 9.64 equiv) was added over 1 min to a solution of tert-butyl 2-methylcarbanilate (515 mg, 2.48 mmol, 4.69 equiv) in THF (3.5 mL) at -40°C. The resulting bright yellow mixture was stirred at -40°C for 15 min, then a solution of the aldehyde, U, (300 mg, 0.529 mmol, 1 equiv) in THF (4 mL) was added. The resulting mixture was warmed to 0°C and was held at that temperature for 15 min. The product solution was diluted with pH 7 phosphate buffer solution (100 mL), and the resulting aqueous mixture was extracted with EtOAc (2x 75 mL). The combined organc layers were dried over Na₂SO₄ and were concentrated. The residue was purified by flash chromatography (5% EtOAc in hexanes initially, grading to 20% ethyl acetate in hexanes) to provide the desired alcohol as a colorless oil (85 mg, 21%) as well as the undesired diastereomeric alcohol as a colorless oil (81 mg, 20%).

¹H NMR (400 MHz, CDCl₃), d 7.78 (br s, 1H, NH), 7.59 (br d, 1H, J=7.9 Hz, ArH), 7.32-7.07 (m, 7H, PhH and ArH), 7.00 (br t, 1H, J=7.3 Hz, ArH), 3.81 (m, 2H, OCH₂ CH₂ O), 3.75 (m, 2H, OCH₂ CH₂ O), 3.67 (s, 1H, (CH₃ CH₂)₃ SiOCH), 3.11 (br d, 1H, J=5.1 Hz, HOCH), 2.77-2.56 (m, 6H, PhCH₂, ArCH₂ and NCH₂), 2.10 (m, 2H, NCH₂ CH (CH₃)₂ and NCH₂ or CH₂), 2.03 (d, 1H, J=11.2 Hz, NCH₂ or CH₂), 1.94 (d, 1H, J=11.4 Hz, NCH₂ or CH₂), 1.84 (m, 2H, NCH₂ CH (CH₃)₂ and NCH₂ or CH₂), 1.71 (m, 3H, NCH₂ and/or CH₂ and NCH₂ CH(CH₃)₂. 1.52 (s, 9H, OC(CH₃)₃), 0.98 (t, 9H, J=7.9 Hz, (CH₃ CH₂)₃ Si), 0.86 (d, 3H, J=6.4 Hz, CH(CH₃)₂), 0.85 (d, 3H, J=6.4 Hz, CH (CH₃)₂), 0.64 (q, 6H, J=7.9 Hz, (CH₃ CH₂)₃ Si).

TLC (20% EtOAc-hexanes), R_{f}: desired alcohol: 0.36 (UV), undesired alcohol: 0.44 (UV)

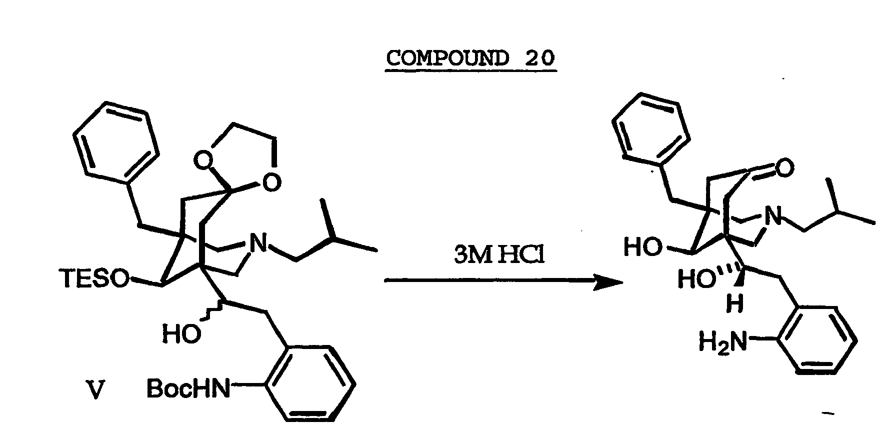

A solution of the ketal (80 mg, 0.11 mmol) in a mixture of aqueous 3M HCl solution (10 mL) and acetone (10 mL) was heated at 60°C. for 16 h. After cooling to 23°C., the product solution was carefully diluted with aqueous saturated NaHCO₃ solution (100 mL). The resulting aqueous mixture was extracted with EtOAc (2x 50 mL). The combined organic layers were dried over Na₂SO₄ and were concentrated. The residue was purified by flash chromatography (40% EtOAc in Hexanes initially, then 40% Hexanes in EtOAc) to afford the product ketone as a colorless oil (31 mg, 67%) . The product oil was triturated with Et₂O to produce a white crystalline solid (mp=164°-165°C.).

¹H NMR (400 MHz, CDCl₃) d 7.32-7.12 (m, 5H, PhH), 7.08 (td, 1H, J=7.6, 1.4 Hz, ArH), 7.00 (dd, 1H, J=7.5, 1.3 Hz, ArH), 6.80 (td, 1H, J=7.5, 1.1 Hz, ArH), 6.71 (dd, 1H, J=7.9, 0.7 Hz, ArH), 3.98 (br s, 1H, OH), 3.88 (br s, 1H, HOCH), 3.74 (br s, 2H, NH), 3.68 (dd, 1H, J=10.4, 1.7 Hz, HOCH), 2.87 (d, 1H, J=15.9 Hz, NCH₂), 2.77 (m, 3H, PhCH₂ and ArCH₂), 2.65 (br d, 1H, J=14.7 Hz, ArCH₂), 2.62 (d, 1H, J=16.1 Hz, NCH₂), 2.46 (m, 2H, NCH₂ CH(CH₃)₂), 2.04 (m, 5H, NCH₂ and CH₂), 1.91 (d, 1H, J=11.5 Hz, NCH₂ or CH₂), 1.61 (m, 1H, NCH₂ CH(CH₃)₂), 0.77 (d, 3H, J=6.6 Hz, CH(CH₃)₂), 0.76 (d, 3H, J=6.4 Hz, CH(CH₃)₂). High-Res MS (FAB): Calcd for C₂₇ H₃₆ N₂ O₃ [M+H]⁺ : 437.2804 Found: 437.2813 Calcd for C₂₇ H₃₆ N₂ O₃ : C, 74.28; H, 8.31; N, 6.42. Found: C, 74.28; H, 8.34; N, 6.52; TLC (40% EtOAc-hexanes), R_{f}:0.06

## Claims

1. Use of a compound of formula (I), or a pharmaceutically acceptable salt thereof: 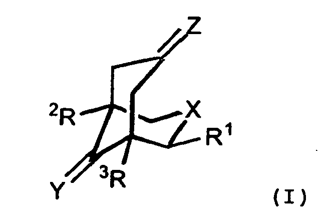 wherein X is --O--, --NH--, --NR⁴-- or --S--;
Y is =O, or forms, with the carbon to which it is attached, 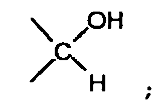 Z is =O, or forms, with the carbon to which it is attached, 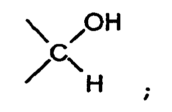 R¹ is
a) H;
b) C₁₋₄ alkyl;
c) C₃₋₇ cycloalkyl;
d) aryl, unsubstituted or substituted one or more times with hydroxy;
e) CH₂R⁵; or
f) 5-7 membered heterocycle; and
R² is
a) C₁₋₄ alkyl;
b) aryl, unsubstituted or substituted with aryl;
c) CH₂R⁶; or
d) heterocycle; and
R³ is
a) CH (OH) R⁷; or
b) CH (NH₂) R⁷; and
R⁴ is
a) C₁₋₄ alkyl;
b) C₃₋₆ cycloalkyl;
c) aryl unsubstituted or substituted with halo or with C₁₋₄ alkyl unsubstituted or substituted one or more times with hydroxy;
d) CH₂R¹; or
e) 5-7 membered heterocycle; and
R⁵ is
a) C₁₋₄ alkyl; or
b) aryl; and
R⁶ is
a) C₁₋₄ alkyl;
b) aryl unsubstituted or substituted with halo or with C₁₋₄ alkyl unsubstituted or substituted one or more times with hydroxy; or
c) 5-7 membered heterocycle; and
R⁷ is
a) H;
b) C₁₋₄ alkyl;
c) aryl unsubstituted or substituted with amino;
d) C₁₋₃ alkylaryl unsubstituted or substituted with amino; or
e) 5-7 membered heterocycle
for the preparation of a medicament for treating a patient who has, or in preventing a patient from getting, a disease or condition selected from the group consisting of Alzheimer's disease, for helping prevent or delay the onset of Alzheimer's disease, for treating patients with mild cognitive impairment (MCI) and preventing or delaying the onset of Alzheimer's disease in those who would progress from MCI to AD, for treating Down's syndrome, for treating humans who have Hereditary Cerebral Hemorrhage with Amyloidosis of the Dutch-Type, for treating cerebral amyloid angiopathy and preventing its potential consequences, i.e. single and recurrent lobar hemorrhages, for treating other degenerative dementias, including dementias of mixed vascular and degenerative origin, dementia associated with Parkinson's disease, dementia associated with progressive supranuclear palsy, dementia associated with cortical basal degeneration, or diffuse Lewy body type of Alzheimer's disease.

2. The use according to claim 1 for the preparation of a medicament for treating or preventing Alzheimer's disease in a patient in need of such treatment.

3. The use according to any of claims 1 or 2, where the medicament further comprises a P-gp inhibitor, or a pharmaceutically acceptable salt thereof.

4. The use according to any of claims 1-3 wherein the compound of formula (I) is selected from the group consisting of:
5(RS)-((4')-2"-furanyl)methylpheny-9(RS)-hydroxy-1(RS)-hydroxy-methyl-3-(2'-methylpropyl-3-azabicyclo[3.3.1]nonan-7-one;
5(RS)-methylphenyl-9(RS)-hydroxy-1(RS)-((1'-hydroxy)-2'-phenyl)-ethyl-3-(2"-methyl)propyl-3-azabicyclo[3.3.1] nonan-7-one;
5(RS)-methylpheny-9(RS)-hydroxy-1(RS)-((1'-hydroxy)-2'-phenyl)-ethyl-3-benzyl-3-azabicyclo[3.3.1]nonan-7-one;
5(RS)-methylpheny-9(RS)-hydroxy-1(RS)-((1'-hydroxy)-2'-(2"'-(tetra-hydro-1,2-thiazine-1,1-dioxide))-ethyl-3-benzyl-3-azabicyclo[3.3.1]nonan-7-one;
5(RS)-methylpheny-9(RS)-hydroxy-1(RS)-((1'-hydroxy)-2'-(2"-amino)-phenyl)-ethyl-3-benzyl-3-azabicyclo[3.3.1]nonan-7-one; 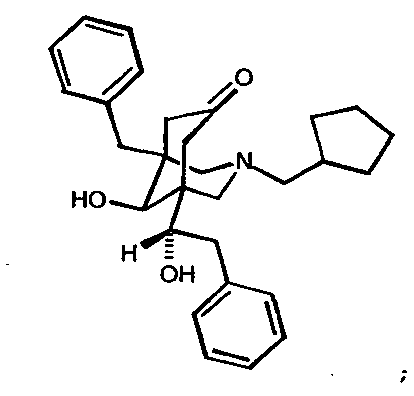 and
compounds 1-23,
and pharmaceutically acceptable salts thereof.

5. The use according to claim 1 where the compound of Formula (I) is contained in a composition comprising one or more pharmaceutically acceptable carriers.

6. The use according to any of claims 1-5, where the medicament further comprises one or more therapeutic agents selected from the group consisting of an antioxidant, an anti-inflammatory, a gamma secretase inhibitor, a neurotrophic agent, an acetyl cholinesterase inhibitor, a statin, an A beta peptide, and an anti-A beta peptide.

7. The use according to any of claims 1-6, where the compound of Formula (I) comprises a compound of Formula (II) or a pharmaceutically acceptable salt thereof: 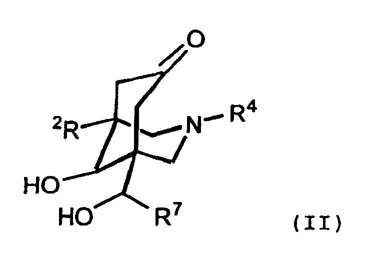 wherein
R² is C₁₋₄ alkylene-aryl; and
R⁴ is C₁₋₄ alkyl, unsubstituted or substituted with aryl, C₃₋₆ cycloalkyl, or 5-7 membered heterocycle;
R⁷ is H, benzyl unsubstituted or substituted with amino.

8. The use according to any of claims 1-3, wherein said treatment is **characterized by** a method comprising administering at the same time, by the same or different route of administration or at different times said medicament together with a P-gp inhibitor, or a pharmaceutically acceptable salt thereof.

9. The use according to any of claims 1-3, wherein said treatment is **characterized by** a method comprising administering at the same time, by the same or different route of administration or at different times said medicament together with on or more therapeutic agents selected from the group consisting of an antioxidant, an anti-inflammatory, a gamma secretase inhibitor, a neurotrophic agent, an acetyl cholinesterase inhibitor, a statin, an A beta peptide, and an anti-A beta peptide.

## Patentansprüche

1. Verwendung einer Verbindung der Formel (I) oder eines pharmazeutisch verträglichen Salzes davon: 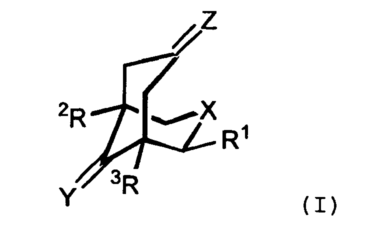 worin X gleich --O--, --NH--, --NR⁴-- oder --S-- ist;
Y gleich =O ist oder bildet mit dem Kohlenstoff, an den es gebunden ist 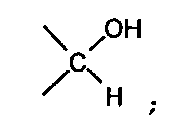 Z gleich =O ist oder bildet mit dem Kohlenstoff, an den es gebunden ist 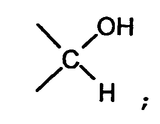 R¹ gleich
a) H;
b) C₁₋₄-Alkyl;
c) C₃₋₇-Cycloalkyl;
d) Aryl, unsubstituiert oder ein- oder mehrfach substituiert mit Hydroxy;
e) CH₂R⁵; oder
f) 5-7-gliedriger Heterozyklus ist; und
R² gleich
a) C₁₋₄-Alkyl;
b) Aryl, unsubstituiert oder mit Aryl substituiert;
c) CH₂R⁶; oder
d) Heterozyklus ist; und
R³ gleich
a) CH(OH)R⁷; oder
b) CH(NH₂)R⁷ ist; und
R⁴ gleich
a) C₁₋₄-Alkyl;
b) C₃₋₆-Cycloalkyl;
c) Aryl, unsubstituiert oder substituiert mit Halogen oder mit C₁₋₄-Alkyl, unsubstituiert oder substituiert ein-oder mehrfach substituiert mit Hydroxy;
d) CH₂R¹; oder
e) 5-7-gliedriger Heterozyklus ist; und
R⁵ gleich
a) C₁₋₄-Alkyl; oder
b) Aryl ist; und
R⁶ gleich
a) C₁₋₄-Alkyl ;
b) Aryl, unsubstituiert oder substituiert mit Halogen oder mit C₁₋₄-Alkyl, unsubstituiert oder ein- oder mehrfach substituiert mit Hydroxy; oder
c) 5-7-gliedriger Heterozyklus ist; und
R⁷ gleich
a) H;
b) C₁₋₄-Alkyl;
c) Aryl, unsubstituiert oder substituiert mit Amino;
d) C₁₋₃-Alkylaryl, unsubstituiert oder substituiert mit Amino; oder
e) 5-7-gliedriger Heterozyklus ist,
zur Herstellung eines Medikaments zur Behandlung eines Patienten, der leidet an, oder zur Prävention, dass ein Patient bekommt, eine Krankheit oder einen Zustand, ausgewählt aus der Gruppe, bestehend aus Alzheimerkrankheit, zur Unterstützung bei der Verhinderung oder dem Verzögern des Auftretens der Alzheimerkrankheit, zur Behandlung von Patienten mit geringer kognitiver Beeinträchtigung (MCI) und zur Verhinderung oder zur Verzögerung des Auftretens der Alzheimerkrankheit in jenen Patienten, die im Krankheitsverlauf von MCI zu AD fortschreiten würden, zur Behandlung des Down-Syndroms, zur Behandlung von Menschen mit hereditärer zerebraler Haemorrhagie mit Amyloidose des Dutch-Typs, zur Behandlung zerebraler amyloider Angiopathie und zur Verhinderung ihrer potenziellen Folgen, nämlich von einmaligen und wiederkehrenden Lobarhaemorrhagien, zur Behandlung anderer degenerativer Demenz, einschließlich Demenz gemischten vaskulären und degenerativen Ursprungs, Demenz im Zusammenhang mit Parkinson, Demenz im Zusammenhang mit progressiver supranukleärer Lähmung, Demenz im Zusammenhang mit kortikaler Basaldegeneration, oder Alzheimerkrankheit des diffusen Lewy-Body-Typs.

2. Verwendung nach Anspruch 1 zur Herstellung eines Medikaments zur Behandlung oder Prävention von Alzheimerkrankheit bei einem Patienten, der eine solche Behandlung benötigt.

3. Verwendung nach einem der Ansprüche 1 oder 2, wobei das Medikament des Weiteren einen P-gp-Inhibitor oder ein pharmazeutisch verträgliches Salz davon umfasst.

4. Verwendung nach einem der Ansprüche 1 bis 3, wobei die Verbindung der Formel (I) ausgewählt ist aus der Gruppe, bestehend aus:
5-(RS)-((4')-2''-Furanyl)methylpheny-9-(RS)-hydroxy-1-(RS)-hydroxymethyl-3-(2'-methylpropyl-3-azabicyclo[3.3.1]nonan-7-on;
5-(RS)-Methylphenyl-9-(RS)-hydroxy-1-(RS)-((1'-hydroxy)-2'-phenyl) ethyl-3- (2"-methyl)propyl-3-azabicyclo[3.3.1]nonan-7-on;
5-(RS)-Methylpheny-9-(RS)-hydroxy-1-(RS)-((1'-hydroxy)-2'-phenyl)ethyl-3-benzyl-3-azabicyclo[3.3.1]nonan-7-on;
5-(RS)-Methylpheny-9-(RS)-hydroxy-1-(RS)-((1'-hydroxy)-2'-(2"'-(tetrahydro-1,2-thiazin-1,1-dioxid))ethyl-3-benzyl-3-azabicyclo[3.3.1]nonan-7-on;
5-(RS)-Methylpheny-9-(RS)-hydroxy-1-(RS)-((1'-hydroxy)-2'-(2"-amino)phenyl)ethyl-3-benzyl-3-azabicyclo[3.3.1]nonan-7-on; 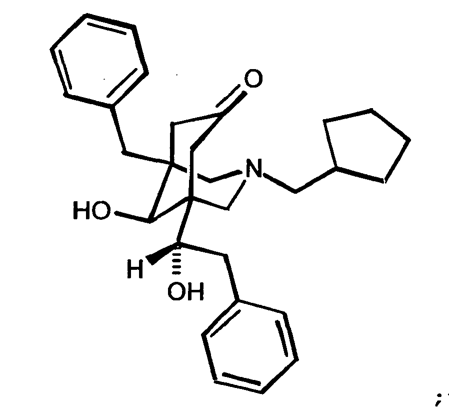 und
Verbindungen 1-23,
und pharmazeutisch verträgliche Salze davon.

5. Verwendung nach Anspruch 1, wobei die Verbindung der Formel (I) in einer Zusammensetzung enthalten ist, umfassend einen oder mehrere pharmazeutisch verträgliche Trägerstoffe.

6. Verwendung nach einem der Ansprüche 1 bis 5, wobei das Medikament des Weiteren ein oder mehrere therapeutische Mittel umfasst, ausgewählt aus der Gruppe, bestehend aus einem Antioxidans, einem entzündungshemmenden Mittel, einem Gammasecretaseinhibitor, einem neurotrophen Mittel, einem Acetylcholinesteraseinhibitor, einem Statin, einem A-Beta-Peptid und einem anti-A-Beta-Peptid.

7. Verwendung nach einem der Ansprüche 1-6, wobei die Verbindung der Formel (I) eine Verbindung der Formel (II) oder ein pharmazeutisch verträgliches Salz davon umfasst: 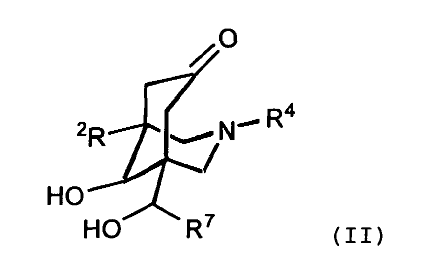 worin
R² C₁₋₄-Alkylen-Aryl ist; und
R⁴ C₁₋₄-Alkyl, unsubstituiert oder substituiert mit Aryl, C₃₋₆₋Cycloalkyl, oder einem 5-7-gliedrigem Heterozyklus ist;
R⁷ H, Benzyl, unsubstituiert oder mit Amino substituiert, ist.

8. Verwendung nach einem der Ansprüche 1-3, wobei die Behandlung **gekennzeichnet ist durch** ein Verfahren umfassend die gleichzeitige Verabreichung des Medikaments **durch** den gleichen oder verschiedene Verabreichungswege oder zu verschiedenen Zeiten, zusammen mit einem P-gp-Inhibitor, oder eines pharmazeutisch verträglichen Salzes davon.

9. Verwendung nach einem der Ansprüche 1-3, wobei die Behandlung **gekennzeichnet ist durch** ein Verfahren umfassend die gleichzeitige Verabreichung des Medikaments **durch** den gleichen oder verschiedene Verabreichungswege oder zu verschiedenen Zeiten, zusammen mit einem oder mehreren therapeutischen Mitteln, ausgewählt aus der Gruppe, bestehend aus einem Antioxidans, einem entzündungshemmenden Mittel, einem Gammasecretaseinhibitor, einem neurotrophen Mittel, einem Acetylcholinesteraseinhibitor, einem Statin, einem A-Beta-Peptid und einem anti-A-Beta-Peptid.

## Revendications

1. Utilisation d'un composé de formule (I), ou d'un sel acceptable sur le plan pharmaceutique de celui-ci : 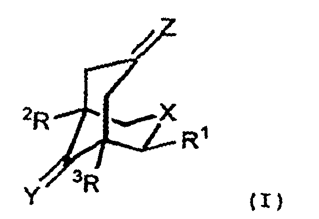 dans laquelle
X est -O-, -NH-, -NR⁴- ou -S- ;
Y est =O ou forme, avec le carbone auquel il est lié, 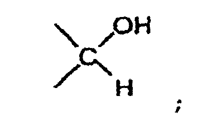 Z est =O ou forme, avec le carbone auquel il est lié, 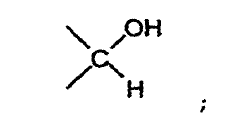 R¹
a) H ;
b) un alkyle en C₁₋₄ ;
c) un cycloalkyle en C₃₋₇ ;
d) un aryle, non substitué ou substitué une ou plusieurs fois par un hydroxyle ;
e) CH₂R⁵ ; ou
f) un hétérocycle à 5-7 éléments ; et
R² est
a) un alkyle en C₁₋₄ ;
b) un aryle, non substitué ou substitué par un aryle ;
c) CH₂R⁶ ; ou
d) un hétérocycle ; et
R³ est
a) CH(OH)R⁷; ou
b) CH(NH₂)R⁷ ; et
R⁴ est
a) un alkyle en C₁₋₄ ;
b) un cycloalkyle en C₃₋₆ ;
c) un aryle non substitué ou substitué par un halogéno ou par un alkyle en C₁₋₄ non substitué ou substitué une ou plusieurs fois par un hydroxyle ;
d) CH₂R¹ ; ou
e) un hétérocycle à 5-7 éléments ; et
R⁵ est
a) un alkyle en C₁₋₄ ; ou
b) un aryle ; et
R⁶ est
a) un alkyle en C₁₋₄ ;
b) un aryle non substitué ou substitué par un halogéno ou par un alkyle en C₁₋₄ non substitué ou substitué une ou plusieurs fois par un hydroxyle ; ou
c) un hétérocycle à 5-7 éléments ; et
R⁷ est
a) H ;
b) un alkyle en C₁₋₄ ;
c) un aryle, non substitué ou substitué par un amino ;
d) un alkyl(en C₁₋₃)aryle non substitué ou substitué par un amino ; ou
e) un hétérocycle à 5-7 éléments ;
pour la préparation d'un médicament pour traiter un patient qui souffre, ou pour la prévention chez un patient, d'une maladie ou d'un état choisi dans le groupe constitué de la maladie d'Alzheimer, pour aider à prévenir ou retarder la survenue de la maladie d'Alzheimer, pour traiter des patients souffrant de troubles cognitifs légers (MCI) et pour prévenir ou retarder la survenue de la maladie d'Alzheimer chez ceux dont les MCI risquent d'évoluer en maladie d'Alzheimer, pour traiter le syndrome de Down, pour traiter les êtres humains qui souffrent d'une hémorragie cérébrale héréditaire avec une amyloïdose de type Dutch, pour traiter l'angiopathie amyloïde cérébrale et prévenir ses éventuelles conséquences, à savoir des hémorragies lobaires occasionnelles et récurrentes, pour traiter d'autres démences dégénératives, dont la démence d'origine à la fois vasculaire et dégénérative, la démence associée à la maladie de Parkinson, la démence associée à la paralysie supranucléaire progressive, la démence associée à la dégénérescence cortico-basale ou le type de maladie d'Alzheimer à corps de Lewy diffus.

2. Utilisation selon la revendication 1 pour la préparation d'un médicament pour traiter ou prévenir la maladie d'Alzheimer chez un patient nécessitant un tel traitement.

3. Utilisation selon l'une quelconque des revendications 1 ou 2, dans laquelle le médicament comprend en outre un inhibiteur de la P-gp, ou un sel acceptable sur le plan pharmaceutique de celui-ci.

4. Utilisation selon l'une quelconque des revendications 1 à 3, dans laquelle le composé de formule (I) est choisi dans le groupe constitué de :
la 5(RS)-((4')-2"-furanyl)méthylphény-9(RS)-hydroxy-1(RS)-hydroxy-méthyl-3-(2'-méthylpropyl-3-azabicyclo[3.3.1]nonan-7-one ;
la 5(RS)-méthylphényl-9(RS)-hydroxy-1(RS)-((1'-hydroxy)-2'-phényl)-éthyl-3-(2"-méthyl)propyl-3-azabicyclo[3.3.1]nonan-7-one ;
la 5(RS)-méthylphény-9(RS)-hydroxy-1(RS)-((1'-hydroxy)-2'-phényl)-éthyl-3-benzyl-3-azabicyclo-[3.3.1]nonan-7-one ;
la 5(RS)-méthylphény-9(RS)-hydroxy-1(RS)-((1'-hydroxy)-2'-(2'''-(tétra-hydro-1,2-thiazine-1,1-dioxyde))-éthyl-3-benzyl-3-azabicyclo[3.3.1]nonan-7-one ;
la 5(RS)-méthylphény-9(RS)-hydroxy-1(RS)-((1'-hydroxy)-2'-(2"-amino)-phényl)-éthyl-3-benzyl-3-azabicyclo[3.3.1]nonan-7-one ; 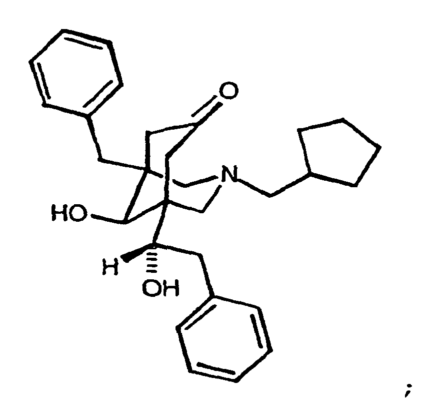 et
les composés 1 à 23,
et leurs sels acceptables sur le plan pharmaceutique.

5. Utilisation selon la revendication 1, dans laquelle le composé de formule (I) est contenu dans une composition comprenant un ou plusieurs véhicules acceptables sur le plan pharmaceutique.

6. Utilisation selon l'une quelconque des revendications 1 à 5, dans laquelle le médicament comprend en outre un ou plusieurs agents thérapeutiques choisis dans le groupe constitué d'un antioxydant, d'un anti-inflammatoire, d'un inhibiteur de la gamma-secrétase, d'un agent neurotrophique, d'un inhibiteur de l'acétylcholinestérase, d'une statine, d'un A bêta-peptide et d'un anti-A bêta-peptide.

7. Utilisation selon l'une quelconque des revendications 1 à 6, dans laquelle le composé de formule (I) comprend un composé de formule (II) ou un sel acceptable sur le plan pharmaceutique de celui-ci : 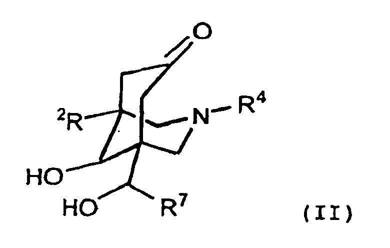 dans laquelle
R² est un alkylène(en C₁₋₄)-aryle ; et
R⁴ est un alkyle en C₁₋₄, non substitué ou substitué par un aryle, un cycloalkyle en C₃₋₆ ou un hétérocycle à 5-7 éléments ;
R⁷ est H, un benzyle non substitué ou substitué par un amino.

8. Utilisation selon l'une quelconque des revendications 1 à 3, dans laquelle ledit traitement est **caractérisé par** un procédé comprenant l'administration simultanée, par une voie d'administration identique ou différente, ou à des moments différents, dudit médicament conjointement avec un inhibiteur de la P-gp, ou un sel acceptable sur le plan pharmaceutique de celui-ci.

9. Utilisation selon l'une quelconque des revendications 1 à 3, dans laquelle ledit traitement est **caractérisé par** un procédé comprenant l'administration simultanée, par une voie d'administration identique ou différente, ou à des moments différents, dudit médicament conjointement avec un ou plusieurs agents thérapeutiques choisis dans le groupe constitué d'un antioxydant, d'un anti-inflammatoire, d'un inhibiteur de la gamma-secrétase, d'un agent neurotrophique, d'un inhibiteur de l'acétylcholinestérase, d'une statine, d'un A bêta-peptide et d'un anti-A bêta-peptide.
